(19) Europäisches Patentamt
European Patent Office
Office européen des brevets

(11) EP 3 950 929 A1

(12) **EUROPEAN PATENT APPLICATION**
published in accordance with Art. 153(4) EPC

(43) Date of publication:
**09.02.2022 Bulletin 2022/06**

(21) Application number: **20779635.0**

(22) Date of filing: **30.03.2020**

(51) International Patent Classification (IPC):
***C12N 1/20*** *(2006.01)* ***A23L 33/105*** *(2016.01)*
***A61K 35/74*** *(2015.01)* ***A61P 35/00*** *(2006.01)*
***C12R 1/01*** *(2006.01)*

(52) Cooperative Patent Classification (CPC):
**A23L 33/105; A61K 35/74; A61P 35/00; C12N 1/20; C12N 1/205;** C12R 2001/01

(86) International application number:
**PCT/KR2020/004363**

(87) International publication number:
**WO 2020/197362 (01.10.2020 Gazette 2020/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.03.2019 KR 20190036156**
**28.03.2019 KR 20190036155**
**25.09.2019 KR 20190118200**

(71) Applicant: **Chunlab, Inc.**
**Seoul 06194 (KR)**

(72) Inventors:
- **KWAK, Min-Jung**
  **Seoul 07278 (KR)**
- **KIM, Byung-Yong**
  **Anyang-si, Gyeonggi-do 14017 (KR)**
- **CHO, Kyoung-Hee**
  **Ansan-si Gyeonggi-do 15301 (KR)**
- **CHOI, Seon-Bin**
  **Gwangju-si, Gyeonggi-do 12768 (KR)**

(74) Representative: **J A Kemp LLP**
**80 Turnmill Street**
**London EC1M 5QU (GB)**

(54) **FAECALIBACTERIUM SP. MICROBE AND ANTICANCER COMPOSITION COMPRISING SAME**

(57) The present invention provides a *Faecalibacterium* spp. microorganism. In addition, it provides a pharmaceutical composition for preventing or treating cancer, or a food composition for preventing or improving cancer, comprising one or more kinds selected from the group consisting of a microbial cell of a *Faecalibacterium cancerinhibens* microorganism having an anti-cancer activity, a culture of the microorganism, a lysate of the microorganism and an extract of the microorganism.

【FIG. 1c】

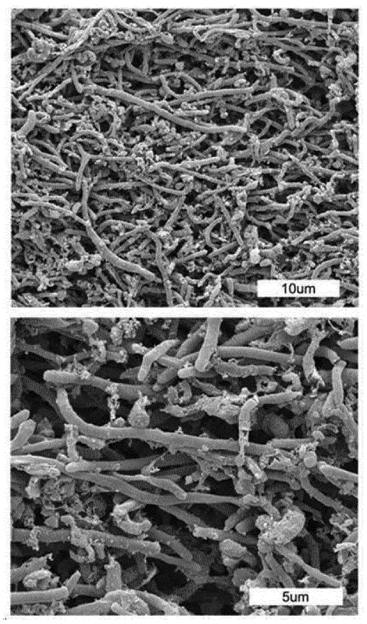


EP 3 950 929 A1

## Description

[TECHNICAL FIELD]

The present invention relates to a *Faecalibacterium* spp. microorganism, and an anti-cancer composition, a pharmaceutical composition for preventing or treating cancer, or a food composition for preventing or improving cancer, comprising the same.

[BACKGROUND ART]

Microbiome means microorganisms that exist in a specific environment and their entire genetic information. In addition to the human body, microbiome information is being used in various fields such as animals, agriculture, marine and environment, and in particular, with the development of human microbiome research based on the development of genetic information analysis and data analysis technology, the growth of the diagnostic industry and healthcare industry based on this is expected.

Human intestinal microorganisms not only break various substances that human enzymes cannot break down and convert them into nutrients that human cells can absorb, but also inhibit the growth of externally derived harmful bacteria to prevent pathogen infection. These intestinal bacteria themselves or various metabolites secreted by the bacteria stimulate immune cells present in the intestinal cells, thereby activating or regulating the immune response of the human body. In addition, due to the number and diversity of symbiotic microorganism genes 100 times greater than that of humans, the human microbiome is also regarded as the second genome of humans and its importance is recognized.

In particular, due to the development of human microbiome research, research results showing that intestinal microorganisms are directly or indirectly related to a number of human diseases are increasing rapidly, and researches showing that intestinal microorganisms are also related to various cancers in the human body are also increasing. Conventional cancer treatment accompanies a combination of chemotherapy, surgery, hormone therapy and/or radiation therapy to remove neoplastic cells of patients. Cancer chemotherapy is preferably based on the use of drugs that kill replicating cells faster than that the agent kills normal cells in patients. Surgery is used to remove the tumor mass, but it has little effect once the cancer has metastasized. Radiation is only effective in localized areas. All these approaches present significant drawbacks and additional risks such as increased susceptibility to infection.

Cancer chemotherapy drugs often cause two complications that require antibiotic treatment, namely, mucositis (a weakened mucosal barrier associated with bacterial translocation) and neutropenia, which can eventually lead to dysbiosis. Thus, there is a strong need for the development of improved cancer therapies that support a constructive interaction between treatment such as chemotherapy and/or radiation and immunity, although it is not a synergistic effect.

[DISCLOSURE]

[TECHNICAL PROBLEM]

An embodiment of the present invention provides a *Faecalibacterium* spp. microorganism having an anti-cancer activity.

An embodiment of the present invention provides an anti-cancer composition, for example, a pharmaceutical composition for preventing or treating cancer, comprising at least one selected from the group consisting of a microbial cell of a *Faecalibacterium* spp. microorganism having an anti-cancer activity; a culture of the microorganism; a lysate of the microorganism; and at least an extract selected from the group consisting of the microorganism, the culture and the lysate.

Other embodiment of the present invention provides an anti-cancer food composition, for example, a food composition for preventing or improving cancer, comprising at least one selected from the group consisting of a microbial cell of a *Faecalibacterium* spp. microorganism having an anti-cancer activity; a culture of the microorganism; a lysate of the microorganism; and at least an extract selected from the group consisting of the microorganism, the culture and the lysate.

[TECHNICAL SOLUTION]

To achieve the objects of the present invention, the present inventors have researched *Faecalibacterium* spp. which is a genus constituting major bacteria in the intestine and have isolated and identified a new microorganism, andmicroorganism and confirmed that the isolated *Faecalibacterium* spp. microorganism had an activity of inhibiting production and proliferation of malignant tumor in a mouse model, thereby completing the present invention.

Hereinafter, the present invention will be described in more detail.

An embodiment of the present invention relates to a *Faecalibacterium* spp. microorganism having an anti-cancer activity, specifically, *Faecalibacterium cancerinhibens,* and more specifically, *Faecalibacterium cancerinhibens* CLCC1. More specifically, the *Faecalibacterium cancerinhibens* according to the present invention is characterized by an anti-cancer activity thereof, for example, an anti-cancer activity against colorectal cancer and/or liver cancer.

Specifically, the *Faecalibacterium* spp. microorganism of the present invention may be isolated from the intestinal microbial flora. The *Faecalibacterium* spp. microorganism having an anti-cancer activity according to the present invention, more specifically, the *Faecalibacterium cancerinhibens* microorganism has at least one characteristic of following characteristics:

(1) an anti-cancer activity, specifically, an activity of preventing cancer occurrence, delaying or inhibiting the growth rate of tumor, reducing the tumor size or volume, or inhibiting or delaying cancer metastasis, for example, an activity of reducing the tumor volume by 10 to 90 % compared to the control group not administered with the microorganism, when *Faecalibacterium cancerinhibens* is administered into mice subcutaneously transplanted with a cancer cell line,
(2) having 16S rRNA comprising a nucleotide sequence of SEQ ID NO: 1 or 16S rRNA comprising a nucleotide sequence having a nucleotide sequence identity of 97% or more, 98% or more, 99% or more, 99.5% or more, 99.8% or more or 99.9% or more,
(3) being cultured at a culture temperature of the microorganism of 20 to 40 °C or under an anaerobic condition,
(4) a lipid production pattern, specifically, not comprising 17:0 iso 30H fatty caid, more specifically, comprising one or more of fatty acids selected from the group consisting of 15:1 ω 8c fatty acid, 19:0 cyclo ω 10*c*/19 ω 6 fatty acid, 20:1 ω 9*c* fatty acid, 14:0 3OH/16:1 iso I fatty acid, 15:0 3OH fatty acid, 16:0 iso fatty acid and 16:0 iso 3OH fatty acid, in particular, comprising PL3 which is not present in *Faecalibacterium prausnitzii,* and
(5) butyric acid production, specifically, having the butyric acid content 2 times to 50 times higher than *Faecalibacterium prausnitzii.*

The *Faecalibacterium* spp. microorganism, for example, *Faecalibacterium cancerinhibens* according to the present invention may have an anti-cancer activity, for example, an activity to prevent cancer occurrence, delay or inhibit the growth rate of tumor, reduce the tumor size or volume, or inhibit or delay cancer metastasis, but not limited thereto. A specific example of the anti-cancer activity may include an activity to inhibit and/or delay cancer metastasis by inhibition of the tumor growth rate. The tumor or cancer includes both gastrointestinal cancers, and non-gastrointestinal cancers, and the non-gastrointestinal cancers include all cancers other than gastrointestinal cancer, and includes, for example, cancer related with the digestive tract system, and the like.

Specifically, it may have an activity to reduce the tumor size, number of cancer cells or growth rate of tumor by at least about 10%, 20%, 30%, 40%, 50%, 60%, 70%, 80%, 90%, 95% or 100%, compared with the corresponding tumor size, number of cancer cells or tumor growth rate in the same subject before treatment, or compared with the corresponding activity in other subject not receiving treatment. In one example of the present invention, the mice transplanted with colorectal cancer cell line which the *Faecalibacterium cancerinhibens* is administered by, showed reduced tumor volume by about 30 to 40 % compared to the mice not administered with the microorganism, thereby confirmed that the effect of the microorganism to reduce the tumor size and inhibit the tumor growth rate in the colorectal cancer.

Herein, "cancer" typically refers to a physiological condition of an animal, having a characteristic of abnormal or uncontrolled cell growth. For example, cancer and cancer pathology may be related to interference with normally functioning surrounding cells, release of cytokines or other secreted products at abnormal levels, inhibition or enhancement of inflammatory or immunological responses, neoplasia, premalignancy, malignancy, surrounding or distant tissues or organs, such as lymph node invasion. Herein, the cancer may be gastrointestinal cancer and non-gastrointestinal cancer, and the non-gastrointestinal cancer is cancer excluding gastrointestinal cancer, and includes cancers related with the digestive tract system, and the like.

The gastrointestinal cancer is a malignant tumor occurring in the gastrointestinal tract system or digestive tract system, for example, the gastrointestinal tract such as esophagus, stomach, small intestine or large intestine and the like, and is found to have advanced to the extent that it is impossible to completely resect the cancer in many cases, because most of them do not have any symptoms, and therefore it belongs to a cancer having poor prognosis. The gastrointestinal cancer or digestive tract system cancer may be one or more cancers selected from the group consisting of esophageal cancer, gallbladder cancer, liver cancer, biliary tract cancer, pancreatic cancer, stomach cancer, small intestine cancer, colorectal cancer, colon cancer, anal cancer and rectal cancer, but not limited thereto, and in one example, it may be colorectal cancer and/or liver cancer.

The non-gastrointestinal cancer includes malignant tumors occurring in tissues other than the gastrointestinal tract system by excluding the gastrointestinal tract, and for example, it may be leukemia, prostate cancer, breast cancer, bladder cancer, kidney cancer, multiple myeloma, cervical cancer, thyroid cancer, ovarian cancer, urethral cancer, osteosarcoma, glioblastoma, brain tumor or lymphoma, but not limited thereto.

More specifically, the anti-cancer activity according to the present invention may be an anti-cancer activity against colorectal cancer and/or liver cancer, and for example, it may be an activity to inhibit occurrence or progress of liver cancer and/or colorectal cancer. Specifically, it may delay tumor occurrence or inhibit the tumor growth rate, and it may further comprise an activity to prevent cancer metastasis due to inhibition of the tumor growth rate.

In one example of the present invention, the liver cancer means all malignant tumors occurred in the liver, and preferably, it may be hepatocellular carcinoma (HCC) or cholangiocarcinoma (CC), and more preferably, it may be one or more cancers selected from the group consisting of hepatoblastoma, cholangiocarcinoma, cholangiocellular cystadenocarcinoma or liver cancer produced by virus infection. The hepatocellular carcinoma is the most important histological subtype, accounting for 70 to 85% of primary liver cancers. In one example of the present invention, the liver cancer may be hepatocellular carcinoma (hepatoma).

In one example of the present invention, the colorectal cancer includes malignant tumors occurring in one or more sites selected from the group consisting of colon ascendens, transverse colon, colon descendens, S-shaped colon and rectal mucous membrane, and for example, it may be colorectal carcinoma. The colorectal cancer may be one or more kinds selected from the group consisting of adenocarcinoma, lymphoma, malignant carcinoid, leimyosarcoma, Kaposi sarcoma and epidermoid carcinoma, but not limited thereto.

The *Faecalibacterium* spp. microorganism according to the present invention is isolated from feces of healthy adult male human, the effect of microorganism to inhibit cancer occurrence and tumor growth in a human cancer cell line, a mouse model subcutaneously transplanted with the cancer cell line, or a mouse model having the corresponding tissue transplanted with the cancer cell line. In one example of the present invention, the effect of inhibiting the tumor growth in the mice induced or transplanted with colorectal cancer or liver cancer. In an example of the present invention, *Faecalibacterium cancerinhibins* strain CLCC1 was isolated from feces of healthy adult males and was confirmed to have an effect of inhibiting tumor growth in mice which was induced for colorectal or liver cancer or transplanted with colorectal or liver cancer.

The *Faecalibacterium* spp. microorganism, for example, *Faecalibacterium cancerinhibens* provided by the present invention may have an activity to inhibit the tumor growth in the cancer cell line or animal model transplanted with cancer cell line, and the inhibition of the tumor growth may delay or inhibit the tumor growth rate or reduce the tumor size or volume. In addition, the microorganism may have an activity to inhibit cancer occurrence or inhibit or delay cancer metastasis. The activity to inhibit the tumor growth may be measured by using for example, the tumor volume/size or tumor weight.

When the *Faecalibacterium cancerinhibens* provided by the present invention is administered to the animal model subcutaneously transplanted with the cancer cell line, compared to the control group not administered by the microorganism, the tumor growth inhibition rate according to the tumor weight may be 5% or more, 10% or more, 15% or more, 20% or more, 25% or more, or 30% or more, and the tumor growth inhibition rate according to the tumor weight may be calculated by following Equation 1.

[Equation 1]

$$IR(\%) = (1-(T1/C1)) \times 100$$

In the Equation 1, IR is the tumor growth inhibition rate according to the tumor weight (IR), and T1 is the average tumor weight in each experimental group, and C1 is the average tumor weight in the negative control group.

When *Faecalibacterium cancerinhibens* strain CLCC1 provided by the present invention is administered to the animal model subcutaneously transplanted with a colorectal cancer cell line, the tumor growth inhibition rate according to the Equation 1 may be 5% or more, 10% or more, 15% or more, 20% or more, 25% or more, or 30% or more, and for example, it may be 5 to 90%, 5 to 70%, 5 to 50%, 10 to 90%, 10 to 70%, 10 to 50%, 15 to 90%, 15 to 70%, 15 to 50%, 20 to 90%, 20 to 70%, 20 to 50%, 25 to 90%, 25 to 70%, 25 to 50%, 30 to 90%, 30 to 70%, or 30 to 50%.

Specifically, in an example of the present invention, the tumor growth inhibition rate was measured using the Equation 1, based on the result of measuring tumor weight for three experimental groups of animal models transplanted with colorectal cancer cell line which are administered with low concentration, medium concentration or high concentration of *Faecalibacterium cancerinhibens* strain CLCC1, and negative control group which are not administered with the microorganism. As a result of measuring the tumor weight according to administration of *Faecalibacterium cancerinhibens* strain CLCC1 in mice subcutaneously transplanted with the human colorectal cancer cell line, it was shown significantly lower all three experimental groups administered with the microorganism, compared to the negative control group. Specifically, it was shown that the tumor growth inhibition rate was 39.6% in the experimental group administered by a low dose of microorganism ($2\times10^6$ cells/head), and the tumor growth inhibition rate was 32.4% in the experimental group administered by a medium concentration of microorganism ($2\times10^7$ cells/head), and the tumor growth inhibition rate was 39.3% in the experimental group administered by at a high concentration of microorganism ($2\times10^8$ cells/head),

hereby confirming that the tumor growth of colorectal cancer was inhibited by 30% or more in all the experimental groups.

When the *Faecalibacterium cancerinhibens* strain CLCC1 provided by the present invention is administered to an animal model subcutaneously transplanted with cancer cell line is, compared to the control group not administered with the microorganism, the relative reduction rate of the tumor volume according to the tumor volume may be % or more, 10% or more, 15% or more, 20% or more, 25% or more, or 30% or more, and the tumor growth inhibition rate according to the tumor volume may be calculated by the following Equation 2.

[Equation 2]

$$\text{Relative reduction rate of tumor volume (\%)} = (1-(T2/C2)) \times 100$$

In the Equation 2, T2 is the average tumor volume in each experimental group and C2 is the average tumor volume in the negative control group.

When *Faecalibacterium cancerinhibens* strain CLCC1 is administered to a tumor animal model transplanted with cancer cell lines according to an example of the present invention, based on 100% of the tumor volume of the negative control group not administered with the microorganism, the tumor volume may be 90% or less, 85% or less, 80% or less, 77% or less, 70% or less, 67% or less, and the tumor volume may have a numerical range combining a value selected from the lower limit of 10% or more, 15% or more, 18% or more, and 20% or more, and a value selected from the upper limit of 90% or less, 85% or less, 80% or less, 77% or less, 70% or less, and 67% or less.

In case that the cancer is colorectal cancer, *Faecalibacterium cancerinhibens* strain CLCC1 may have an activity to reduce the tumor volume by a level of 10 to 90%, 10 to 85%, 10 to 80%, 10 to 77%, 10 to 70%, 10 to 67%, 20 to 90%, 20 to 80%, 20 to 70%, 20 to 67%, 30 to 90%, 30 to 80%, 30 to 70%, 30 to 67%, 40 to 90%, 40 to 80%, 40 to 70%, 40 to 67%, 50 to 90%, 50 to 80%, 50 to 70%, 50 to 67% or 60 to 67%, based on 100% of the tumor volume of the negative control group not administered with strain CLCC1, when strain CLCC1 is administered.

Specifically, the average tumor size was all the same of 105mm$^3$ in all 4 groups at the day (Day 1) when the administration of the microorganism was started in a tumor mouse model subcutaneously transplanted with a human colorectal cancer cell line. However, in 36 days, the volume of 3000mm$^3$ or less was in all the experimental groups administered with the strain CLCC1, whereas the average tumor volume was 4330mm$^3$ in the negative control group. Therefore, they had a tumor volume of about 60 to 67% compared to the control group, thereby confirmed that the effect of *Faecalibacterium cancerinhibens* strain CLCC1 on the growth inhibition of colorectal cancer.

In case that the cancer is liver cancer, *Faecalibacterium cancerinhibens* strain CLCC1 provided by the present invention may have an activity to reduce the tumor volume by a level of 90% or less, 85% or less, 80% or less, 77% or less, 10 to 90%, 10 to 85%, 10 to 80%. 10 to 77%, 15 to 90%, 15 to 85%, 15 to 80%, 15 to 77%, 18 to 90%, 18 to 85%, 18 to 80%, or 18 to 77%, based on 100% of the liver tumor volume of the control group not administered with the strain CLCC1, when the strain CLCC1 was administered.

In the tumor volume reduction of liver cancer according to the present invention, the volume of the liver cancer was 19.5% to 75.3%, and the average volume of the liver cancer was lower by about 32.9% in the experimental groups orally administered with strain CLCC1, compared to the control group. Thus, it can be found that the orally administered strain CLCC1 microorganism has an activity to inhibit occurrence and progress of tumor in the liver cancer.

In an example of the present invention, as a result of detecting the occurrence and growth rate of liver cancer using MR imaging, for preparing the mice model administered by *Faecalibacterium cancerinhibens* strain CLCC1 and the control mice not being administered by strain CLCC1, and subcutaneously transplanting them with the cancer cell lines, the tumor volume of liver cancer showed lower values by 32.9% on average compared to the control mice. Therefore, it confirmed the delay of cancer occurrence and delay of the tumor growth progress rate by the microorganism.

In one example of the present invention, as a result of comparting the tumor size (volume) of the experimental groups administered with the *Faecalibacterium cancerinhibens* strain CLCC1 microorganism and the control group not administered, after causing liver cancer by transplanting a liver cancer cell line, Hep55.1c, to a left lobe of liver in 8-week-old mice, the tumor growth was significantly inhibited in the experimental groups administered with the *Faecalibacterium cancerinhibens* strain CLCC1, compared to the control group, when the tumor size on the day of transplantation was set to 1.

In one example of the present invention, when the mice was treated to have liver cancer by a subcutaneous transplantation with liver cancer cell line and was orally administered by *Faecalibacterium cancerinhibens* strain CLCC1 in the process of liver cancer occurrence for 55 days, it was confirmed that the tumor occurrence and progress were statistically significantly inhibited in the experimental groups orally administered with *Faecalibacterium cancerinhibens* strain CLCC1, compared to the control group (FIGs. 4a to 4c). On average, the tumor volume was about 19.5% to 75.3%, and the average tumor volume was lower by 32.9% in the experimental groups orally administered by strain CLCC1, compared to the control group. Thus, it can be found that the orally administered strain CLCC1 has an activity to inhibit the tumor occurrence and progress.

In one example of the present invention, when the mice was treated to have liver cancer by liver transplantation and was orally administered by *Faecalibacterium cancerinhibens* strain CLCC1 in the process of liver cancer occurrence for 39 days, it was confirmed that the cancer occurrence and progress were statistically significantly inhibited in the experimental groups orally administered with strain CLCC1, compared to the control group not administered with strain CLCC1 (FIG. 5a to FIG. 5c). Therefore, it can be confirmed that the strain CLCC1 has an excellent activity of inhibition of tumor occurrence and growth.

The *Faecalibacterium* spp. microorganism, for example, *Faecalibacterium cancerinhibens* according to the present invention may be a microorganism having 16S rRNA having a nucleotide sequence of SEQ ID NO: 1 or 16S rRNA comprising a nucleotide sequence having a nucleotide sequence identity of 97% or more, 98% or more, 99% or more, 99.5% or more, 99.8% or more or 99.9% or more.

The *Faecalibacterium cancerinhibens* according to the present invention may be cultured at a temperature of 20 to 40 °C, 50 to 40 °C, 30 to 40 °C, 35 to 40 °C or 37°C, and it may be characterized by being cultured under an anaerobic condition.

The *Faecalibacterium cancerinhibens* according to the present invention may have a characteristic showing a lipid production pattern different from *Faecalibacterium prausnitzii* which is a *Faecalibacterium* spp. microorganism.

The *Faecalibacterium cancerinhibens* according to the present invention does not comprise 17:0 iso 3OH fatty acid. In one example of the present invention, as a result of analyzing the polar lipid content of *Faecalibacterium cancerinhibens* strain CLCC1 and *Faecalibacterium prausnitzii,* it was confirmed that strain CLCC1 additionally had PL3 which is absent in *Faecalibacterium prausnitzii* (FIG. 3b). The *Faecalibacterium cancerinhibens* according to the present invention may comprise one or more fatty acids selected from the group consisting of 15:1 ω8*c* fatty acid, 19:0 cyclo ω10*c*/19ω6 fatty acid, 20:1 ω9*c* fatty acid, 14:0 3OH/16:1 iso I fatty acid, 15:0 3OH fatty acid, 16:0 iso fatty acid and 16:0 iso 3OH fatty acid.

In one example of the present invention, as a result of comparing the fatty acid composition of *Faecalibacterium cancerinhibens* strain CLCC1 and *Faecalibacterium prausnitzii* by performing gas chromatography analysis, there was a difference in the composition of major fatty acids between two microorganisms. Specifically, 15:1 ω8*c* fatty acid, 19:0 cyclo ω10*c*/19ω6 fatty acid, 20:1 ω9*c* fatty acid, 14:0 3OH/16:1 iso I fatty acid, 15:0 3OH fatty acid, 16:0 iso fatty acid and 16:0 iso 3OH fatty acid were detected only in strain CLCC1 , but 17:0 iso 3OH fatty acid was detected only in *Faecalibacterium prausnitzii* ATCC 27768.

The *Faecalibacterium cancerinhibens* provided by the present invention has the high production of butyric acid among short chain fatty acids (SCFA). Specifically, the butyric acid production of *Faecalibacterium cancerinhibens* strain CLCC1 of the present invention may be 2 to 50 times, 2 to 40 times, 2 to 30 times, 2 to 20 times, 2 to 15 times, 2 to 10 times, 5 to 50 times, 5 to 40 times, 5 to 30 times, 5 to 20 times, 5 to 15 times, or 5 to 10 times, compared to *Faecalibacterium prausnitzii.* Specifically, as a result of SCFA analysis of the culture supernatant of *Faecalibacterium cancerinhibens* strain CLCC1 and *Faecalibacterium prausnitzii,* the butyric acid content of *Faecalibacterium cancerinhibens* strain CLCC1 showed 7.8 times higher than that of *Faecalibacterium prausnitzii* (FIGs. 3c to 3d).

The preferable *Faecalibacterium* spp. microorganism according to the present invention was deposited to Biological Resource Center of Korea Research Institute of Bioscience and Biotechnology on January 3, 2019 and received an accession number of KCTC 13783BP, and the scientific name is *Faecalibacterium cancerinhibens.*

The *Faecalibacterium cancerinhibens* strain CLCC1 according to the present invention forms a colony within 3mm or within 2mm and has a long rod-shaped cell shape with an observation by optical microscope, when smearing and culturing on an agar medium. In one example of the present invention, *Faecalibacterium cancerinhibens* strain CLCC1 had a genome size of 2.9Mbp, and the number of protein coding regions (CDS) was 2695, and the GC ratio was 56.1% and the number of rRNA genes was 21, and the total number of tRNA genes was 69. In *Faecalibacterium cancerinhibens* strain CLCC1 according to the present invention, the butyric acid (butanoic acid) at a content 7.8 times higher than *Faecalibacterium prausnitzii* was detected (FIGs. 3c to 3d).

An embodiment of the present invention provides an anti-cancer composition, for example, a pharmaceutical composition for preventing or treating cancer, comprising at least one selected from the group consisting of a microbial cell of a *Faecalibacterium* spp. microorganism having an anti-cancer activity; a culture of the microorganism; a lysate of the microorganism; and an extract of at least one selected from the group consisting of the microorganism, culture and lysate. Specifically, the cancer includes gastrointestinal cancer and non-gastrointestinal cancer, and for example, it may be colorectal cancer and/or liver cancer.

The composition of the present invention is used for prevention, treatment or improvement of cancer. The composition of the present invention may be administered to a patient who is diagnosed as cancer or confirmed to have a risk of cancer occurrence. The example of the present invention proved that the administration of the composition of the present invention can reduce the tumor growth in various tumor models. The treatment of cancer using the composition of the present invention may further have an activity to decrease or reduce metastasis of cancer cells in addition to the activity of delaying tumor formation and/or tumor growth of cancer cells.

The *Faecalibacterium* spp. microorganism, for example, *Faecalibacterium cancerinhibens* strain CLCC1 according to the present invention used in the anti-cancer composition is same as described above.

The anti-cancer agent according to the present invention may be used alone or together with other therapies, for example, surgery, radiation therapy, gene therapy, immunotherapy (e.g., target antibody immunotherapy, CAR-T cytotherapy), oncolytic virus, bone marrow transplantation, stem cell transplantation, hormone therapy, target treatment, cryotherapy, ultrasonic treatment, photodynamic therapy, chemotherapy or the like. In addition, a person at high risk of developing a proliferative disease may receive treatment of inhibiting and/or delaying an occurrence of the disease. The anti-cancer activity of the composition according to the present invention may be more effective when combined with a direct anti-cancer agent. Thus, in a specific embodiment, the present invention provides a composition comprising *Faecalibacterium cancerinhibens* strain CLCC1 and an anti-cancer agent.

The active ingredient of the anti-cancer composition according to the present invention, one or more kinds selected from the group consisting of a microbial cell of a *Faecalibacterium* spp. microorganism having an anti-cancer activity; a culture of the microorganism; a lysate of the microorganism; and an extract of one or more kinds selected from the group consisting of the microorganism, culture and lysate may be comprised in an amount of 0.00001 % by weight to 100 % by weight, 0.001 % by weight to 99.9 % by weight, 0.1 % by weight to 99 % by weight, more preferably, 1 % by weight to 50 % by weight in the total anti-cancer composition.

The *Faecalibacterium* spp. microorganism may comprise a microbial cell itself or be a cell-free form not comprising a microbial cell. The lysate means a lysate obtained by crushing the *Faecalibacterium* spp. microbial cells or a supernatant obtained by centrifuging the lysate. Herein, unless mentioned otherwise, the *Faecalibacterium* spp. microorganism having an anti-cancer activity is used to mean one or more kinds selected from the group consisting of a microbial cell of the microorganism; a culture of the microorganism; a lysate of the microorganism; and an extract of one or more kinds selected from the group consisting of the microorganism, culture and lysate.

The anti-cancer composition according to the present invention may comprise a lyophilized microbial cell. The lyophilization of the microbial cell of the microorganism may be performed with a method known to those skilled in the art. Alternatively, the composition of the present invention may comprise a culture of a live microorganism. In one embodiment, the microorganism contained in the anti-cancer composition according to the present invention is not inactivated, and for example, it is not heat-inactivated. In some embodiments, the microorganism contained in the anti-cancer composition according to the present invention is not killed, for example, not heat-killed. In some embodiments, the microorganism contained in the anti-cancer composition according to the present invention is not weakened, for example, not heat-weakened. In some embodiments, the microorganism contained in the anti-cancer composition according to the present invention may colonize the intestine partially or entirely.

The anti-cancer composition according to the present invention comprises the microorganism of the present invention in a therapeutically effective amount. The microorganism in a therapeutically effective amount is sufficient for exhibiting a beneficial effect to a patient. The therapeutically effective amount of the bacterial strain may be sufficient to cause the intestinal transport and/or partial or entire colonization of a patient. For example, an appropriate daily dose of the bacteria for an adult human may be about $1 \times 10^3$ to about $1 \times 10^{11}$ colony forming unit (CFU); for example, about $1 \times 10^7$ to about $1 \times 10^{10}$ CFU; and in other example, it is about $1 \times 10^6$ to about $1 \times 10^{10}$ CFU.

The pharmaceutical composition according to the present invention may be administered to mammals including humans through various routes. The administration method may be all the commonly used methods, and for example, it may be administered through an oral, intradermal, intravenous, intramuscular or subcutaneous route, and preferably, it may be orally administered.

The composition of the present invention may comprise a pharmaceutically acceptable excipient or carrier. The carrier or diluent acceptable for therapeutic use is well known in the pharmaceutical field. The example of the appropriate carrier includes lactose, starch, glucose, methyl cellulose, magnesium stearate, mannitol, sorbitol, and the like. The example of the diluent includes ethanol, glycerol and water. The selection of the pharmaceutical carrier, excipient or diluent may be conducted regarding the intended administration route and standard pharmaceutical practice. The pharmaceutical composition may comprise any appropriate binding agent, lubricant, suspending agent, coating agent or solubilizing agent, as the carrier, excipient or diluent, or in addition to them.

The dose of the pharmaceutical composition of the present invention may vary depending on the patient’s age, body weight, gender, administration type, health condition and disease degree, and it may be administered in divided doses from once to several times a day at a certain time interval depending on the judgement of the doctor or pharmacist. For example, based on the active ingredient content, the daily dose may be 0.1 to 500 mg/kg, preferably, 0.5 to 300 mg/kg. The above dose is an example of an average case, and the dose may be higher or lower depending on individual differences.

The composition of the present invention may be a probiotic, and the probiotic may be mixed with one appropriate prebiotic compound. The prebiotic compound is generally oligo- or polysaccharide or non-digestive carbohydrate such as sugar alcohol, and this is not degraded or absorbed in the upper digestive tract. The known prebiotic includes commercially available products such as inulin and transgalacto-oligosaccharide.

The composition of the present invention may be encapsulated to transport the *Faecalibacterium* spp. microorganism in the intestine. The encapsulation protects the composition until transported to the target site, for example, through rupture by chemical or physical stimuli, such as physical disruption that may be caused by changes in pressure, enzymatic activity or pH. Any appropriate encapsulation method may be used. An illustrative encapsulation technology includes capture in porous matrix, attachment or adhesion on the surface of a solid carrier, aggregation or autoagglutination by a crosslinking agent, and mechanical blockage after microporous membranes or microcapsules.

One example of the present invention relates to an anti-cancer composition, for example, a food composition or anti-cancer functional food composition for preventing or improving cancer, comprising a microbial cell of a *Faecalibacterium* spp. microorganism having an anti-cancer activity; a culture of the microorganism; a lysate of the microorganism; and an extract of one or more kinds selected from the group consisting of the microorganism, culture and lysate.

The *Faecalibacterium* spp. microorganism, for example, *Faecalibacterium cancerinhibens* strain CLCC1 according to the present invention used in the anti-cancer composition is same as described above.

Herein, the food means a natural substance or processed product containing one or more nutrients, and preferably, refers to a state that may be directly eaten through a certain amount of processing process, and is intended to include all foods, food additives, health functional foods, beverages and beverage additives, and the like in a conventional sense. Herein, the beverage means a generic term for drinking to quench thirst or enjoy taste and is intended to include functional beverages. The beverage may be a liquid, syrup and/or gel form.

As an active ingredient contained in the food composition, the content of the *Faecalibacterium* spp. microorganism is not particularly limited, suitably depending on the type of food, desired use, and the like, and for example, one or more kinds selected from the group consisting of a microbial cell of the *Faecalibacterium* spp. microorganism, a culture of the microorganism, a lysate of the microorganism and an extract of the microorganism which are active ingredients of the total food weight may be added in an amount of 0.00001 % by weight to 100 % by weight, 0.001 % by weight to 99.9 % by weight, 0.1 % by weight to 99 % by weight, more preferably, 1 % by weight to 50 % by weight, 0.01 to 15 % by weight. For example, the food composition may be added at a ratio of 0.02 to 10 g, preferably, 0.3 to 1 g, based on 100 Mℓ.

The composition of the present invention may be formulated. For example, food may provide nutritional benefits in addition to the therapeutic effect of the present invention as a nutritional supplement. Similarly, the food composition may be formulated to enhance the taste of the composition of the present invention or to make it more attractive for consumption by making it more like common food items rather than the pharmaceutical composition.

[ADVANTAGEOUS EFFECTS]

The *Faecalibacterium* spp. microorganism and a composition for preventing or treating cancer comprising the same according to the present invention has the activity to inhibit the occurrence and growth of cancer, and in particular, it is ingested by humans in a form of a pharmaceutical composition or health functional food, or the like, and therefore, it can be used for prevention, treatment and/or improvement of cancer.

[BRIEF DESCRIPITON OF THE DRAWINGS]

FIG. 1a is a photograph showing the colony form formed after culturing *Faecalibacterium cancerinhibens* strain CLCC1 in Reinforced clostridial media (RCM) for 3 days.

FIG. 1b is a photograph observing the cell appearance with an optical microscope in an exponential growth phase during the liquid culture of *Faecalibacterium cancerinhibens* strain CLCC1.

FIG. 1c is a photograph taken with a scanning electron microscope of cells in an exponential growth phase during the liquid culture of *Faecalibacterium cancerinhibens* strain CLCC1.

FIG. 2a is a diagram showing the phylogenetic position obtained through 16S rRNA analysis of *Faecalibacterium cancerinhibens* strain CLCC1.

FIG. 2b is a diagram showing the phylogenetic position using 92 core genes present in the genome of *Faecalibacterium cancerinhibens* strain CLCC1.

FIG. 2c is a relationship diagram of *Faecalibacterium cancerinhibens* strain CLCC1 strain and related species based on average nucleotide identity (ANI) values.

FIG. 2d is a table comparing ANI values of *Faecalibacterium cancerinhibens* strain CLCC1 and related species.

FIG. 3a is a graph of the result of gas chromatography analysis comparing the fatty acid composition of *Faecalibacterium cancerinhibens* strain CLCC1 and *Faecalibacterium prausnitzii* strain.

FIG. 3b is a photograph showing the result of two-dimensional chromatography analysis performed for comparison of polar lipids of *Faecalibacterium cancerinhibens* strain CLCC1 and *Faecalibacterium prausnitzii* strain.

FIG. 3c is a graph of GC-MS analysis of short-chain fatty acid analysis of *Faecalibacterium cancerinhibens* strain CLCC1.

FIG. 3d is a graph of GC-MS analysis performing on short-chain fatty acid analysis of *Faecalibacterium prausnitzii*.

FIG. 4a to FIG. 4c are graphs showing the change in the tumor volume with time after inducing liver cancer to measure the inhibitory effect of cancer occurrence of *Faecalibacterium cancerinhibens* strain CLCC1 in mice induced with liver cancer by the method of Example 4. FIG. 4a is a dot graph showing each subject with gum, and FIG. 4b is a graph of comparing average values of the experimental group and the control group, and FIG. 4c is a polygonal graph showing the volume change of the tumor for each subject, respectively.

FIG. 5a to FIG. 5c are test results of the inhibitory effect of cancer occurrence of *Faecalibacterium cancerinhibens* strain CLCC1 in a liver transplantation model and dot graphs and polygonal graphs of each result. FIG. 5a means the result of comprising tumor sizes directly and FIG. 5b means the result of representing the change in tumor sizes with a fold, and FIG. 5c means a delta value.

FIG. 6a is a graph showing the change in tumor volume according to the dose of *Faecalibacterium cancerinhibens* strain CLCC1 in mice subcutaneously transplanted with a human colorectal cancer cell line.

FIG. 6b is a graph showing the result of measuring the tumor weights according to the dose of *Faecalibacterium cancerinhibens* strain CLCC1 in mice subcutaneously transplanted with a human colorectal cancer cell line.

FIG. 6c is the result of visually observing the change in tumor sizes according to the dose of *Faecalibacterium cancerinhibens* strain CLCC1 in mice subcutaneously transplanted with a human colorectal cancer cell line.

FIG. 6d is the result of isolating and visually observing the tumor tissue in the negative control group (G1) and each experimental group (G2 to G4) after the end of the administration period of *Faecalibacterium cancerinhibens* strain CLCC1 in mice subcutaneously transplanted with a human colorectal cancer cell line.

FIG. 6e is the result of performing H&E staining to confirm cell necrosis according to administration of *Faecalibacterium cancerinhibens* strain CLCC1 in the tumor tissue of mice subcutaneously transplanted with a human colorectal cancer cell line.

FIG. 6f is the result of performing TUNEL staining to confirm apoptosis according to administration of *Faecalibacterium cancerinhibens* strain CLCC1 in the tumor tissue of mice subcutaneously transplanted with a human colorectal cancer cell line.

FIG. 7 is the result of observing the change in tumor sizes according to administration of *Faecalibacterium cancerinhibens* strain CLCC1 in the tumor tissue of mice subcutaneously transplanted with a human colorectal cancer cell line.

FIG. 8 shows an experimental design for administering various test agents in an animal model having cancer in order to experiment the anti-cancer efficacy according to one example of the present invention.

[MODE FOR INVENTION]

Hereinafter, the present invention will be described in more detail by examples. However, the scope of the present invention is not limited by the following examples.

**Example 1. Isolation of microorganism**

*Faecalibacterium* spp. bacteria are the dominant bacteria in the intestine of healthy adults, accounting for about 10% of the total intestinal bacteria, and are known to directly or indirectly affect health.

*Faecalibacterium cancerinhibens* strain CLCC1 was isolated by diluting and applying feces of a healthy 20s adult male in a reinforced clostridial media (RCM) medium and then culturing at a room temperature of 37 °C under an anaerobic condition.

*Faecalibacterium cancerinhibens* strain CLCC1 has a characteristic of forming colonies of 2 to 3mm at maximum and showing a long rod-shaped cell shape when observed with an optical microscope, when cultured in an RCM agar medium for 3 days. In FIGs. 1a and 1b, the result of observing the colony form and the cell shape using an optical microscope was shown. In FIG. 1c, a photograph taking the cell shape with a scanning electron microscope was shown.

**Example 2. Phylogenetic analysis of microorganism**

**2-1. Genome sequencing**

*Faecalibacterium cancerinhibens* strain CLCC1 isolated by the method of Example 1 was cultured in an RCM liquid medium at a room temperature of 37 °C under an anaerobic culture condition for 1 day, and then centrifugation was performed to obtain a microorganism. Using FastDNA SPIN Kit for Soil (MP Biomedicals), the total genome was isolated from the obtained microorganism.

After producing a sequencing library using PacBio sequencing library kit, the total genome sequencing was performed using PacBio RS II platform, and using Whole Genome analysis pipeline of EzBioCloud, our genome data analysis platform, the genome sequencing information was analyzed.

In Table 1 below, the genome characteristics of *Faecalibacterium cancerinhibens* strain CLCC1 were shown. It was shown that the total genome size of *Faecalibacterium cancerinhibens* strain CLCC1 was about 2.9Mbp, and the number of protein coding regions (CDS) was 2695, and the GC ratio (%) was 56.1%. It was shown that the total rRNA genes were 21 and the total tRNA genes were 69.

[Table 1]

| Item | Value |
|---|---|
| Genome size (bp) | 2,941,967 |
| Number of protein coding regions (Number of CDSs) | 2,695 |
| GC ratio (%) | 56.1 |
| Number of rRNA genes | 21 |
| Number of tRNA genes | 69 |

### 2-2. Phylogenetic analysis using 16S rRNA analysis

Phylogenetic analysis was performed by analyzing rRNA of *Faecalibacterium cancerinhibens* strain CLCC1 sequenced by the method of Example 2-1. The 16S rRNA sequence of *Faecalibacterium cancerinhibens* strain CLCC1 was shown in SEQ ID NO: 1.

Specifically, the similarity analysis of 16S rRNA was performed using 16S-based identification for prokaryote pipeline of EzBioCloud database, and the phylogenetic analysis was conducted using MEGA program. In FIG. 2a, the phylogenetic position of *Faecalibacterium cancerinhibens* strain CLCC1 isolated by analyzing the 16S rRNA gene nucleotide sequence was shown.

As the result of the 16S rRNA similarity analysis, *Faecalibacterium cancerinhibens* strain CLCC1 exhibited 16S rRNA similarity of 98% to *Faecalibacterium prausnitzii* ATCC 27768(T). The phylogenetic position of strain CLCC1 is "*Firmicutes* phylum, *Clostridia* class, *Clostridiales* order, *Ruminococcaceae* family".

### 2-3. Phylogenetic analysis using core genes

Phylogenetic analysis was performed through analysis between gene sequences using the genome sequence data obtained by the method of Example 2-1.

Specifically, phylogenetic analysis was conducted using similarity of 92 genes (up-to-date bacterial core gene (UBCG)) which could be used as a taxonomic marker of bacteria. The 92 genes refer to genes provided by UBCG (up-to-date bacterial core gene) pipeline (Na, S. I., Kim, Y. O., Yoon, S. H., Ha, S. M., Baek, I. & Chun, J. (2018)).

In FIG. 2b, a diagram showing phylogenetic positions with allied species obtained by analyzing 92 core genes was represented.

### 2-4. Relationship analysis using Average Nucleotide Identity (ANI)

Using the genome analysis data obtained by the method of Example 2-1, the relationship was analyzed by comparing ANI values of *Faecalibacterium cancerinhibens* strain CLCC1 and allied species.

Specifically, using Genome-based identification for prokaryote (TrueBAC ID) pipeline, an analysis platform provided by Chunlab, the genome similarity values were analyzed by comparing the total genome sequence of *Faecalibacterium cancerinhibens* strain CLCC1 and allied species.

In FIG. 2c, the relationship diagram of *Faecalibacterium cancerinhibens* strain CLCC1 and allied species based on ANI values was shown. In FIG. 2d, the table of comparison of ANI values of *Faecalibacterium cancerinhibens* strain CLCC1 and allied species was shown. In each row and column, 1 means *Faecalibacterium cancerinhibens* strain CLCC1, and 2 means *Faecalibacterium prausnitzii* ATCC 27768(T), and 3 means Fournierella massiliensis AT2(T), and 4 means Intestinimonas butyriciproducens DSM 26588(T), and 5 means Intestinimonas massiliensis GD2(T), and 6 means Pseudoflavonifractor capillosus ATCC 29799(T), and 7 means Ruthenibacterium lactatiformans 585-1(T), and 8 means Subdoligranulum variabile DSM 15176(T), and 9 means Gemmiger formicilis ATCC 27749(T). (T) after each specific name means a type strain.

As the result of decoding the genome of *Faecalibacterium cancerinhibens* strain CLCC1 and comparing with allied species, the strain CLCC1 showed an average nucleotide identity (ANI) value of 85.99% at a genome level with Faecalibacterium prausnitzil which is the closest allied species at the total genome level, and it can be found that it is a novel species not isolated and reported conventionally. The *Faecalibacterium* spp. strain identified by the above method was named *Faecalibacterium cancerinhibens* CLCC1.

**Example 3. Analysis of fatty acid content of microorganism**

To analyze the molecular biological characteristics of *Faecalibacterium cancerinhibens* strain CLCC1, the fatty acid composition of *Faecalibacterium cancerinhibens* strain CLCC1 and *Faecalibacterium prausnitzii* was compared.
Specifically, *Faecalibacterium cancerinhibens* strain CLCC1 strain and *Faecalibacterium prausnitzii* ATCC 27768 were cultured in an RCM medium at a temperature of 37°C under an anaerobic condition, respectively, and then using microbial cells of 40mg, fatty acids were obtained according to the method of Miller (Miller, L. T. (1982) J. Clin. Microbiol. 18, 861-867).
In analysis of the fatty acids extracted, Agilent technologies 6890 Gas chromatography was used, and as a separation column, A30m × 0.320mm × 0.25μm Crosslinked Methyl siloxane column (HP-1) was used. In FIG. 3a, a graph of the result of gas chromatography analysis of comparing the fatty acid composition of *Faecalibacterium cancerinhibens* strain CLCC1 and *Faecalibacterium prausnitzii* ATCC 27768 was shown. In the graph of FIG. 3a, the names of fatty acids that each peak means in the graph of FIG. 3a and their values (%, w/v) were shown in Table 2 below.

[Table 2]

| Fatty acid type | Content in CLCC1 (%) | Content in ATCC 27768 (%) |
|---|---|---|
| 16:00 | 32.42 | 42.28 |
| 18:1 ω7 | 28.15 | 14.88 |
| 17:0 2OH | 12.51 | 8.09 |
| 16:1 ω7*c*/16:1 ω6*c* | 5.22 | 6.04 |
| 16:1 ω9*c* | 3.89 | 1.57 |
| 14:00 | 2.44 | 14.93 |
| 17:0 anteiso | 2.42 | 0.99 |
| 16:1 ω5*c* | 2.17 | 0.83 |
| 18:1 ω9*c* | 1.88 | 0.86 |
| 18:00 | 1.59 | 2.17 |
| 16:1 2OH | 1.41 | 0.51 |
| 20:1 ω7*c* | 0.84 | 0.43 |
| 18:1 ω5*c* | 0.81 | 0.32 |
| 15:0 2OH | 0.75 | 0.5 |
| 18:0 10-methyl, TBSA | 0.45 | 0.21 |
| 15:1 ω8*c* | 0.44 | - |
| 15:1 iso H/13:0 3OH | 0.35 | 1.44 |
| 16:0 3OH | 0.32 | 0.11 |
| 15:0 anteiso | 0.21 | 0.2 |
| 12:00 | 0.2 | 1.59 |
| 15:0 iso 3OH | 0.17 | 0.49 |
| 17:00 | 0.17 | 0.2 |
| 19:0 cyclo ω10*c*/19ω6 | 0.16 | - |
| 20:00 | 0.14 | 0.1 |

(continued)

| Fatty acid type | Content in CLCC1 (%) | Content in ATCC 27768 (%) |
|---|---|---|
| 13:1 at 12-13 | 0.13 | 0.35 |
| 10:00 | 0.13 | 0.2 |
| 20:1 ω9*c* | 0.13 | - |
| 17:0 iso | 0.12 | 0.17 |
| 14:0 3OH/16:1 iso I | 0.12 | - |
| 15:0 3OH | 0.11 | - |
| 16:0 iso | 0.1 | - |
| 16:0 iso 3OH | 0.05 | - |
| 17:0 iso 3OH | - | 0.54 |

As shown in the Table 2, 15:1 ω8*c* fatty acid, 19:0 cyclo ω10*c*/19ω6 fatty acid, 20:1 ω9*c* fatty acid, 14:0 3OH/16:1 iso I fatty acid, 15:0 3OH fatty acid, 16:0 iso fatty acid and 16:0 iso 3OH fatty acid were detected only in strain CLCC1, and 17:0 iso 3OH fatty acid was detected only in *Faecalibacterium prausnitzii* ATCC 27768.

As the result of comparison analysis of the molecular biological characteristics, it could be confirmed that *Faecalibacterium cancerinhibens* strain CLCC1 was a different species from *Faecalibacterium prausnitzii* which is a known species included in *Faecalibacterium* spp, . When comparing the fatty acid composition of two microorganisms, it could be found that there was a difference in the composition of major fatty acids between two microorganisms, and the corresponding result was shown in FIG. 3a and Table 2.

**Example 4. Polar lipid analysis of microorganism**

To compare the polar lipid composition, thin layer chromatography (TLC) analysis was performed.

Specifically, the polar lipid was extracted from a microorganism lyophilized sample of 50 mg by the method of Minikin (Minikin, D.E., et al. (1984). J Microbial Meth 2, 233-241.). In addition, the primary development was performed in a solvent having a ratio of chloroform, methanol and water of 65:25:3.8(v/v) (chloroform:methanol:water = 65:25:3.8(v/v)). Then, secondary development was formed in a solvent having a ratio of chloroform, methanol, acetate and water of 40:7.5:6:1.8(v/v) (chloroform:methanol:acetic acid:water = 40:7.5:6:1.8(v/v)). After the secondary development, it was strained with 5%(w/v) ethanolic molybdatophosphoric acid.

In addition, when comparing the composition of the polar lipid, it was confirmed that *Faecalibacterium cancerinhibens* strain CLCC1 and *Faecalibacterium prausnitzii* ATCC 27768 had one phosphatidylglycerol (PG), two unknown unidentified phospholipids (PL1, PL2), and a plurality of unidentified lipids (L). In addition, it was confirmed that strain CLCC1 had unidentified phospholipids (PL3) not present in *Faecalibacterium prausnitzii* ATCC 27768 additionally, and the corresponding result was shown in FIG. 3b. As could be confirmed from the result, it can be found that the polar lipid production patterns of *Faecalibacterium cancerinhibens* strain CLCC1 and *Faecalibacterium prausnitzii* are different.

**Example 5. Short chain fatty acid (SCFA) analysis**

To examine the difference of short chain fatty acid production patterns of *Faecalibacterium prausnitzii* ATCC 27768 and *Faecalibacterium cancerinhibens* strain CLCC1, by requesting analysis to Korea Research Institute of Bio medical Science, the analysis of the short chain fatty acid distribution was performed.

Specifically, after culturing *Faecalibacterium prausnitzii* ATCC 27768 and *Faecalibacterium cancerinhibens* strain CLCC1 of the present invention in an RCM medium for 16 hours, cells were precipitated by centrifugation (4°C, 4,000 rpm, 10min) to collect the supernatant. The collected supernatant was refrigerated after filtering with a 0.22um filter. A GC-MS sample for SCFA analysis from the supernatant was prepared using the method of Takeshi Furuhashi (Takeshi Furuhashi, et al., Analytical Biochemistry, Volume 543, 2018, Pages 51-54), and 6890 series equipment of Agilent was used for the short chain fatty acid analysis using GC-MS.

As the result of comparison analysis of short chain fatty acids (SCFA) present in the culture supernatant of *Faecalibacterium cancerinhibens* strain CLCC1 and *Faecalibacterium prausnitzii* ATCC 27768, other known species of *Faecalibacterium* spp., in both samples, as SCFA, only butyric acid (butanoic acid) was detected. In addition, butyric acid (butanoic acid) at a content 7.8 times higher was detected in the *Faecalibacterium cancerinhibens* strain CLCC1 sample, compared to the *Faecalibacterium prausnitzii* ATCC 27768 sample. In FIGs. 3c to 3d, the graph of the GC-MS analysis was represented.

Furthermore, when looking at the ratio of butyric acid in each sample, 91.64% of the culture supernatant of *Faecalibacterium cancerinhibens* strain CLCC1 was butyric acid, and 76.40% of the culture supernatant of *Faecalibacterium prausnitzii* ATCC 27768 was butyric acid. The result of the short chain fatty acid analysis of *Faecalibacterium cancerinhibens* strain CLCC1 was shown in Table 3, and the result of the short chain fatty acid analysis of *Faecalibacterium prausnitzii* ATCC 27768 was shown in Table 4. Other substances indicated in Table 3 to Table 4 below were detected in the substance library database, but they were classified as noise signals that could not accurately identify the substances because of their very low matching rates (quality). In Table 3 below, RT means a retention time.

[Table 3]

| Peak | RT | Library/ID | Matching rate (Quality) | Area | Ratio (% of total, %) |
|---|---|---|---|---|---|
| 1 | 2.6965 | Oxirane | 58 | 606,058 | 3.132 |
| 2 | 3.1915 | Butanoic acid (CAS); n-Butyric acid | 94 | 17,732,33 7 | 91.635 |
| 3 | 3.5174 | Ethane, methoxy- (CAS); Methane | 50 | 497,085 | 2.569 |
| 4 | 4.1933 | 2-Pentanone, 4-hydroxy-4-methyl- (CAS) | 43 | 515,638 | 2.665 |

[Table 4]

| Peak | RT | Library/ID | Matching rate (Quality) | Area | Ratio (% of Total, %) |
|---|---|---|---|---|---|
| 1 | 2.6908 | 2-Decanone (CAS) | 38 | 338,372 | 11.491 |
| 2 | 3.0167 | Butanoic acid (CAS); n-Butyric acid | 93 | 2,249,717 | 76.401 |
| 3 | 4.2057 | 2-Pentanone, 4-hydroxy-4-methyl- (CAS) | 47 | 356,542 | 12.108 |

Butyric acid is known to have an anti-cancer activity, and it can be predicted that *Faecalibacterium cancerinhibens* strain CLCC1 of the present invention exhibits an excellent anti-cancer activity, due to higher production of butyric acid among short chain fatty acids, compared to *Faecalibacterium prausnitzii* ATCC 27768.

**Example 6. Test of inhibitory effect of cancer occurrence in subcutaneous tumor model using mouse liver cancer cell line**

To confirm the inhibiting ability of cancer occurrence of *Faecalibacterium cancerinhibens* strain CLCC1, the inhibitory effect of cancer occurrence was tested in a subcutaneous tumor model using a mouse liver cancer cell line. In 10 6-week-age male C57BL/6 mice, through a subcutaneous tumor transplantation experiment of a liver cancer cell line (Hep55.1c), liver cancer was caused, and during a process of liver cancer occurrence for 55 days, the effect of oral administration of *Faecalibacterium cancerinhibens* strain CLCC1 on the growth of liver cancer cells was examined. The liver cancer cell line Hep55.1c was mouse-derived hepatocellular carcinoma (hepatoma).

Specifically, *Faecalibacterium cancerinhibens* strain CLCC1 was orally administered 4 days before the mouse liver cancer cell line was transplanted into the mouse subcutaneous tissue. At Day 4 after oral administration, the mouse liver cancer cell line (Hep55.1c) was transplanted into the subcutaneous tissue of the right flank to make $2.0 \times 10^6$ cells.

For the subcutaneous tumor model using the mouse liver cancer cell line, *Faecalibacterium cancerinhibens* strain CLCC1 was orally administered once a day, 5 times a week, for 55 days. Then, from the day of transplantation (Day 0) to Day 55, the size of the liver cancer tumor (tumor volume) was measured using Magnetic Resonance (MR) imaging at a 7-day interval.

*Faecalibacterium cancerinhibens* strain CLCC1 was cultured in an RCM medium, and then concentrated and stored frozen using a PBS buffer and glycerol to a final glycerol concentration of 15%(v/v). In addition, after thawing immediately before administration to mice, 200ul ($2\times10^8$cells) per animal was orally administered. As a control group, 10 mice not administered with strain CLCC1 were used. For the control group, 200ul each of PBS and glycerol (15%, v/v) solution containing no strain was orally administered.

In Table 5 and Table 6 below, the result of the tumor size of the control group and test group in the test of the inhibitory effect of liver cancer occurrence of the isolated strain in the subcutaneous tumor model was shown. Table 5 is the result of the tumor volume change for the control group, and Table 6 is the result of the tumor size (volume) change of the experimental group administered with *Faecalibacterium cancerinhibens* strain CLCC1. In the tables, S.D means standard deviation.

[Table 5]

| | Control | | | | | | | | | | | |
|---|---|---|---|---|---|---|---|---|---|---|---|---|
| Day | 1 | 2 | 3 | 4 | 5 | 6 | 7 | 8 | 9 | 10 | Mean | S.D. |
| 0 | 0.0 | 0.0 | 0.0 | 0.0 | 0.0 | 0.0 | 0.0 | 0.0 | 0.0 | 0.0 | 0.0 | 0.00 |
| 4 | 31.2 | 1.3 | 5.8 | 14.3 | 5.5 | 2.9 | 0.8 | 2.6 | 5.0 | 20.0 | 8.9 | 9.90 |
| 7 | 47.4 | 18.2 | 27.1 | 13.2 | 12.6 | 16.2 | 13.8 | 13.2 | 19.3 | 23.9 | 20.5 | 10.64 |
| 10 | 132.0 | 55.4 | 74.7 | 51.4 | 79.7 | 81.7 | 70.4 | 50.0 | 76.5 | 101.1 | 77.3 | 24.80 |
| 13 | 132.3 | 81.9 | 100.2 | 115.5 | 147.2 | 86.8 | 73.5 | 55.6 | 99.0 | 184.3 | 107.6 | 38.31 |
| 17 | 160.0 | 115.9 | 120.0 | 99.4 | 144.7 | 126.8 | 101.3 | 124.7 | 102.6 | 182.9 | 127.8 | 27.29 |
| 20 | 236.7 | 223.2 | 121.8 | 173.4 | 206.3 | 137.6 | 136.4 | 119.0 | 165.8 | 348.9 | 186.9 | 70.61 |
| 24 | 282.6 | 261,1 | 124.6 | 250.0 | 303.4 | 249.0 | 185.1 | 160.0 | 203.7 | 388.3 | 240.8 | 76.37 |
| 27 | 547.9 | 393.6 | 205.3 | 302.7 | 527.5 | 323.3 | 262.7 | 252.5 | 294.1 | 469.5 | 357.9 | 120.33 |
| 31 | 425.0 | 513.5 | 323.3 | 338.6 | 488.8 | 341.2 | 323.0 | 307.8 | 312.7 | 554.8 | 392.9 | 94.30 |
| 34 | 571.6 | 570.2 | 402.5 | 489.1 | 611.4 | 387.2 | 366.2 | 410.1 | 380.5 | 728.3 | 491.7 | 123.16 |
| 38 | 562.0 | 586.1 | 502.6 | 517.2 | 819.3 | 557.2 | 529.1 | 509.2 | 493.4 | 819.3 | 589.5 | 124.50 |
| 41 | 563.2 | 902.2 | 574.0 | 673.2 | 761.5 | 506.4 | 626.6 | 659.6 | 604.4 | 1008.7 | 688.0 | 158.98 |
| 45 | 815.8 | 1009.7 | 883.2 | 759.6 | 1222.4 | 721.7 | 806.3 | 753.0 | 656.1 | 1101.4 | 873.5 | 181.41 |
| 48 | 904.1 | 784.4 | 1035.6 | 772.8 | 1074.4 | 735.2 | 726.5 | 802.0 | 701.6 | 1306.9 | 884.4 | 196.55 |
| 52 | 1039.7 | 1413.8 | 1031.2 | 1128.0 | 1146.8 | 940.3 | 981.9 | 939.7 | 807.5 | 1623.9 | 1105.3 | 243.74 |
| 55 | 1098.8 | 2037.5 | 1201.0 | 1327.7 | 1636.2 | 1076.8 | 993.3 | 961.8 | 930.0 | 1813.3 | 1307.6 | 389.62 |

[Table 6]

| | CLCC1 | | | | | | | | | | | | |
|---|---|---|---|---|---|---|---|---|---|---|---|---|---|
| Day | 1 | 2 | 3 | 4 | 5 | 6 | 7 | 8 | 9 | 10 | Mean | S.D. | p-value |
| 0 | 0.0 | 0.0 | 0.0 | 0.0 | 0.0 | 0.0 | 0.0 | 0.0 | 0.0 | 0.0 | 0.0 | 0.00 | |
| 4 | 5.5 | 4.3 | 0.9 | 0.0 | 2.1 | 4.3 | 1.8 | 0.0 | 0.8 | 2.6 | 2.2 | 1.93 | |
| 7 | 24.8 | 16.0 | 10.2 | 3.6 | 7.7 | 19.7 | 15.4 | 14.0 | 1.8 | 9.0 | 12.2 | 7.14 | |
| 10 | 86.5 | 59.6 | 52.3 | 19.5 | 57.5 | 64.4 | 72.1 | 60.7 | 12.7 | 60.8 | 54.6 | 22.42 | |
| 13 | 96.6 | 74.9 | 89.9 | 65.2 | 63.6 | 101.3 | 94.6 | 119.4 | 48.9 | 78.3 | 83.3 | 21.01 | 0.0949 |
| 17 | 121.6 | 99.8 | 112.6 | 76.9 | 103.9 | 122.5 | 89.3 | 116.4 | 112.9 | 73.1 | 102.9 | 17.87 | 0.0266 |
| 20 | 164.0 | 169.8 | 160.7 | 63.1 | 121.3 | 190.0 | 83.9 | 135.3 | 63.9 | 101.1 | 125.3 | 45.90 | 0.0328 |
| 24 | 226.0 | 173.4 | 165.6 | 101.3 | 125.4 | 302.6 | 150.2 | 147.3 | 114.4 | 131.4 | 163.8 | 60.16 | 0.0221 |
| 27 | 245.2 | 290.1 | 214.1 | 101.3 | 149.3 | 317.7 | 222.5 | 208.4 | 145.1 | 113.3 | 200.7 | 72.13 | 0.0024 |
| 31 | 315.9 | 412.5 | 363.9 | 216.1 | 229.7 | 436.9 | 218.3 | 371.0 | 245.6 | 280.6 | 309.0 | 82.98 | 0.0491 |
| 34 | 388.7 | 326.8 | 338.0 | 137.4 | 238.1 | 547.0 | 301.6 | 397.3 | 288.7 | 246.6 | 321.0 | 110.22 | 0.0043 |
| 38 | 516.4 | 353.8 | 470.6 | 508.0 | 206.2 | 822.4 | 336.8 | 436.1 | 308.2 | 311.8 | 427.0 | 170.79 | 0.0257 |
| 41 | 683.5 | 494.5 | 448.9 | 470.2 | 382.2 | 1000.9 | 414.0 | 632.5 | 461.5 | 430.2 | 542.3 | 187.73 | 0.0775 |
| 45 | 618.5 | 466.7 | 538.2 | 655.2 | 468.3 | 1058.5 | 462.1 | 712.4 | 246.4 | 484.0 | 571.0 | 214.54 | 0.0032 |
| 48 | 919.4 | 485.3 | 717.5 | 970.2 | 393.3 | 1233.6 | 558.0 | 682.7 | 262.8 | 556.6 | 677.9 | 293.19 | 0.0809 |
| 52 | 936.4 | 441.7 | 789.0 | 1347.2 | 596.2 | 1265.5 | 623.3 | 1063.4 | 548.1 | 620.4 | 823.1 | 315.46 | 0.0381 |
| 55 | 1049.2 | 494.9 | 941.1 | 1369.1 | 595.1 | 1068.7 | 573.9 | 718.0 | 384.5 | 472.9 | 766.8 | 323.14 | 0.0033 |

In FIG. 4, after inducing cancer in mice, the tumor volume according to the time change was shown as a graph, and in the Tables 5 to 6, the change in the tumor volume was shown as numerical values.

As the experimental result, it was confirmed that the liver cancer occurrence and progress were statistically significantly inhibited in the experimental group orally administered with strain CLCC1, compared to the control group. The tumor volume was averagely about from 19.5% to 75.3%, on average, 32.9% lower, in the experimental group orally administered with strain CLCC1, compared to the control group, and thereby it was confirmed that in the experimental group administered with *Faecalibacterium cancerinhibens* strain CLCC1 , the liver cancer occurrence was delayed, and the tumor growth progress rate was inhibited.

In addition, at Day 4 which is the first day of measuring the tumor size after surgery, it can be confirmed that there is an inhibitory effect of cancer occurrence, as the tumor size is definitely small in the experimental group administered with *Faecalibacterium cancerinhibens* strain CLCC1, compared to the control group.

Thus, it can be found that orally administered strain CLCC1 has an activity to inhibit occurrence and progress of liver cancer.

**Example 7. Test of inhibitory effect of cancer occurrence in liver transplantation model using mouse liver cancer cell line**

To examine the effect of *Faecalibacterium cancerinhibens* strain CLCC1 on the growth of liver cancer cells, in 4 8-week-age male C57BL/6 mice, through a liver transplantation experiment of a liver cancer cell line, liver cancer was caused, and during a process of liver cancer occurrence for 39 days, the effect of oral administration of *Faecalibacterium cancerinhibens* strain CLCC1 on the growth of liver cancer cells was examined.

Specifically, as the liver cancer cell line, Hep55.1c was used, and the epigastrium of the 8-week-old male C57BL/6 mice was opened, and the liver cancer cell line was transplanted into the left lobe of liver so as to be $5.0 \times 10^5$ cells. After a recovery period of 4 days after transplanting the tumor cell line into the liver, the tumor size was confirmed by MRI scanning, and 4 mice in which tumor was well formed were selected and used for a subsequent experiment.

For 39 days from the day of transplantation (Day 0), strain CLCC1 was prepared by the same method as Example 4, and orally administered to 4 mice that completed the transplantation operation, and strain CLCC1 was not administered to 4 control mice. For the control group, 200ul each of PBS and glycerol (15%, v/v) solution containing no strain was orally administered. Thereafter, the tumor size and relative size and relative value of growth were periodically measured.

The tumor size was measured by magnetic resonance (MR) imaging at a 7-day interval, and the relative value of the tumor size was drawn by calculating a relative size to the tumor size at the day of transplantation. In FIG. 5a to FIG. 5c, the test result of the inhibitory effect of cancer occurrence of strain CLCC1 in the liver transplantation model and the graph were shown.

FIG. 5a is a dot graph and a polygonal graph which directly compare the tumor size, and FIG. 5b is dot and polygonal graphs representing the change in the tumor size by fold, and FIG. 5c is dot and polygonal graphs representing the change amount of the tumor size (difference between the tumor size on the day of measurement and the tumor size on the day of previous measurement).

As a result, it was confirmed that the liver cancer occurrence and progress were statistically significantly inhibited in the experimental group orally administered with strain CLCC1, compared to the control group not administered with the isolated line. The result for inhibition of liver cancer occurrence and data of the change in the tumor size were shown in FIGs. 5a to 5c.

The relative figure table (Relative growth; Fold) of the tumor size was shown in Table. 7, and the relative figure table (Relative growth; Delta) of the tumor growth is shown in Table 7.

[Table 7]

| | Control | | | | CLCC1 | | | |
|---|---|---|---|---|---|---|---|---|
| **Days after administmtion** | **1** | **2** | **3** | **4** | **1** | **2** | **3** | **4** |
| **0** | 1.0 | 1.0 | 1.0 | 1.0 | 1.0 | 1.0 | 1.0 | 1.0 |
| **4** | 2.0 | 2.4 | 2.6 | 2.8 | 2.2 | 2.2 | 1.4 | 2.2 |
| **11** | 5.8 | 3.1 | 3.7 | 6.5 | 3.9 | 4.2 | 2.5 | 3.5 |
| **18** | 9.5 | 1.0 | 2.1 | 6.8 | 1.6 | 3.0 | 1.0 | 1.8 |
| **25** | 11.3 | 0.5 | 2.1 | 10.8 | 0.9 | 2.9 | 0.5 | 0.7 |

(continued)

| | Control | | | | CLCC1 | | | |
|---|---|---|---|---|---|---|---|---|
| **Days after administmtion** | **1** | **2** | **3** | **4** | **1** | **2** | **3** | **4** |
| **32** | 14.6 | 0.0 | 2.2 | 17.4 | 1.2 | 6.0 | 0.0 | 0.0 |
| **39** | 15.8 | 0.0 | 3.1 | 25.7 | 2.0 | 7.3 | 0.0 | 0.0 |

[Table 8]

| | Control | | | | CLCC1 | | | |
|---|---|---|---|---|---|---|---|---|
| **Days after administration** | **1** | **2** | **3** | **4** | **1** | **2** | **3** | **4** |
| **0** | 0 | 0.0 | 0.0 | 0.0 | 0.0 | 0.0 | 0.0 | 0.0 |
| **4** | 1.0 | 1.4 | 1.6 | 1.8 | 1.2 | 1.2 | 0.4 | 1.2 |
| **11** | 1.9 | 0.3 | 0.4 | 1.3 | 0.8 | 0.9 | 0.8 | 0.7 |
| **18** | 0.6 | -0.7 | -0.4 | 0.0 | -0.6 | -0.3 | -0.6 | -0.5 |
| **26** | 0.2 | -0.5 | 0.0 | 0.6 | -0.4 | -0.0 | -0.5 | -0.6 |
| **32** | 0.3 | -1.0 | 0.0 | 0.6 | 0.3 | 1.1 | -1.0 | -1.0 |
| **39** | 0.1 | 0.0 | 0.4 | 0.5 | 0.7 | 0.2 | 0.0 | 0.0 |

As can be confirmed in Table 7 and Table 8 and FIGs. 5a to 5b, it can be confirmed that the tumor growth of the mice orally administered with strain CLCC1 was remarkably inhibited, compared to the control group. Thus, it can be confirmed that *Faecalibacterium cancerinhibens* strain CLCC1 has an excellent inhibitory ability of tumor growth.

### Example 8. Confirmation of anti-cancer effect in mouse subcutaneous tumor model using human colorectal cancer cell line

#### 8-1. Experimental method for confirmation of anti-cancer effect

To confirm the inhibitory ability of cancer occurrence of *Faecalibacterium cancerinhibens* strain CLCC1, the inhibitory effect of cancer occurrence in a subcutaneous tumor model using a human colorectal cancer cell line was tested. As the colorectal cancer cell line, HCT-116 cells were used, and as the mouse, nude mice (CAnN.Cg-Foxnlnu/CrlOri, SPF) were used. The HCT-116 is a cancer cell line corresponding to human-derived colorectal carcinoma.

Specifically, the colorectal cancer cell line HCT-116 ($2.5\times10^7$ cells/mL) was administered to the mice that had undergone a one-week purification period and transplanted by administering 0.2 mL/head subcutaneously to the right back of mice using a disposable syringe.

When the size of cancer cells grew to about 85~119mm$^3$ after cancer cell transplantation, 10 mice per group were classified into a negative control group not administered with strain CLCC1 and 3 experimental groups administered with strain CLCC1 by concentration. Specifically, the experimental group was divided into 3 groups of low, medium and high according to the concentration of strain CLCC1, and *F. cancerinhibens* strain CLCC1 was administered at a concentration of $2\times10^6$ cells/head for the low concentration, $2\times10^7$ cells/head for the medium concentration, and $2\times10^8$ cells/head for the high concentration. The administration of the strain was oral administration once a day for 36 days with a syringe. For the negative control group, only an excipient (PBS solution comprising glycerol 15%(v/v)) was administered.

In Table 9 below, the information of the negative control group and each experimental group was shown.

[Table 9]

| Mark | CLCC1 dose | Administration solution amount | Description | Number of subj ects (number of animals) |
|---|---|---|---|---|
| G1 | 0 | 0.2mL/head | Negative control group | 10 |

(continued)

| Mark | CLCC1 dose | Administration solution amount | Description | Number of subj ects (number of animals) |
|---|---|---|---|---|
| G2 | $2\times10^6$(cells/ head) | 0 .2mL/head | Low concentration experimental group | 10 |
| G3 | $2\times10^7$(cells/ head) | 0 .2mL/head | Medium concentration experimental group | 10 |
| G4 | $2\times10^8$(cells/ head) | 0 .2mL/head | High concentration experimental group | 10 |

### 8-2. Tumor size change and growth inhibition rate according to microorganism administration

To confirm the anti-colorectal cancer effect of *F. cancerinhibens* strain CLCC1, the change in the tumor size was confirmed. Specifically, once a day after starting the test of strain administration of Example 7-1, general symptoms such as appearance, behavior, excretion and the like were observed, and the body weight was measured once a week, and the tumor size was measured, and the volume was calculated twice a week. In FIGs. 6c and 6d, the result of visually observing the tumor size was shown.

The tumor volume (Tv) was calculated by the method of Equation 3 below by measuring the perpendicular width (W) and maximum length (L) of tumor, respectively.

[Equation 3]

$$Tv\ (mm^3) = L\ (mm) \times W^2\ (mm^2) \times 1/2$$

The result of confirming the change in the tumor volume throughout 36 days was shown in Table 10 and FIG. 6a below. It was confirmed that the tumor size was statistically significantly reduced in all the experimental groups administered with strain CLCC1, compared to the negative control group (G1). In Table 10 below, Mean refers to an average volume and S.D. means standard deviation.

[Table 10]

| Group/ Dose (cells/head) | | Tumor volume ($mm^3$) | | | | | | | | | | |
|---|---|---|---|---|---|---|---|---|---|---|---|---|
| | | Time after administration (days) | | | | | | | | | | |
| | | 1 | 4 | 8 | 11 | 15 | 18 | 22 | 25 | 29 | 32 | 36 |
| G1 | Mean | 105 | 186 | 316 | 520 | 968 | 1331 | 1809 | 2228 | 2748 | 3399 | 4330 |
| 0 | S.D. | 10 | 15 | 26 | 39 | 87 | 145 | 136 | 251 | 347 | 497 | 763 |
| G2 | Mean | 105 | 187 | 304 | 479 | 802 | 1029 | 1269 | 1629 | 1974 | 2408 | 2651 |
| $2\times10^5$ | S.D. | 9 | 17 | 30 | 56 | 127 | 238 | 347 | 572 | 744 | 944 | 931 |
| | | | | | | .. | .. | .. | . | . | . | .. |
| G3 | Mean | 105 | 193 | 312 | 497 | 812 | 1032 | 1306 | 1705 | 2078 | 2481 | 2875 |
| $2\times10^4$ | S.D. | 9 | 20 | 32 | 48 | 143 | 216 | 303 | 544 | 750 | 921 | 1007 |
| | | | | | | .. | .. | .. | . | | . | .. |
| G4 | Mean | 105 | 191 | 303 | 452 | 754 | 926 | 1112 | 1487 | 1821 | 2210 | 2621 |
| $2\times10^8$ | S.D. | 9 | 16 | 40 | 53 | 104 | 201 | 263 | 317 | 471 | 607 | 701 |
| | | | | | .. | .. | .. | .. | .. | .. | .. | .. |

* p<0.05, Significant difference from the negative control group (G1) by Dunnett's t-test.
** p<0.01, Significant difference from the negative control group (G1) by Dunnett's t-test.

Specifically, the average tumor size at the day (Day 1) of staring administration of the microorganism was same

as $105mm^3$ in all the 4 groups, but in 36 days, the average tumor volume was shown as $4330mm^3$ in the negative control group, and on the other hand, the volume of $3000mm^3$ or less was shown in all the experimental groups administered with strain CLCC1, and therefore, it was confirmed that it had the tumor volume of about 60 to 67%, compared to the control group, and thereby, the inhibitory effect of the colorectal cancer growth of the *Faecalibacterium cancerinhibens* strain CLCC1 was confirmed.

In addition, based on the result of weight measurement, the tumor growth inhibition rate (IR) was calculated by the method of Equation 1 below in each experimental group.

[Equation 1]

$$IR(\%) = (1-(T1/C1)) \times 100$$

In the Equation 1, T is the average tumor weight in each experimental group, and C is the average tumor weight in the negative control group.

In Table 11 and FIG. 6b below, the result of measuring the tumor weight in each group after the end of the experiment of administration of the strain was shown, and in Table 12 below, the result of measuring the average body weight of mice of each group according to the progress of the experiment was shown.

[Table 11]

| Group/Dose (cells/head) | | Tumor weights (g) | IR (%) |
|---|---|---|---|
| G1<br>0 | Mean<br>S.D. | 3.21<br>0.83 | 0.0 |
| G2<br>$2\times10^6$ | Mean<br>S.D. | 1.94<br>0.59<br>.. | 39.6 |
| G3<br>$2\times10^7$ | Mean<br>S.D. | 2.17<br>1.02<br>.. | 32.4 |
| G4<br>$2\times10^8$ | Mean<br>S.D. | 1.95<br>0.61<br>.. | 39.3 |
| ** p<0.01, Significant difference from the negative control group (G1) by Dunnett's t-test | | | |

[Table 12]

| Group/ Dose (cells/ head) | | Body weights (g) | | | | | |
|---|---|---|---|---|---|---|---|
| | | Time after administration (days) | | | | | |
| | | 1 | 8 | 15 | 22 | 29 | 36 |
| G1<br>0 | Mean<br>S.D.<br>N | 19.5<br>1.1<br>10 | 19.4<br>1.4<br>10 | 19.4<br>1.5<br>10 | 19.3<br>1.8<br>10 | 19.8<br>1.8<br>10 | 19.4<br>1.7<br>10 |
| G2<br>$2\times10^6$ | Mean<br>S.D.<br>N | 20.3<br>1.3<br>10 | 20.0<br>1.2<br>10 | 19.8<br>1.5<br>10 | 18.9<br>1.1<br>10 | 19.0<br>1.4<br>10 | 18.8<br>1.3<br>10 |
| G3<br>$2\times10^7$ | Mean<br>S.D.<br>N | 20.3<br>0.8<br>10 | 19.5<br>0.9<br>10 | 19.3<br>1.3<br>10 | 18.5<br>1.5<br>10 | 18.9<br>1.4<br>10 | 18.8<br>1.3<br>10 |
| G4<br>$2\times10^8$ | Mean<br>S.D. | 19.8<br>1.2 | 19.3<br>1.4 | 19.1<br>1.5 | 18.7<br>1.4 | 18.5<br>1.5 | 18.4<br>1.6 |

(continued)

| Group/ Dose (cells/ head) | Body weights (g) | | | | | |
|---|---|---|---|---|---|---|
| | Time after administration (days) | | | | | |
| | 1 | 8 | 15 | 22 | 29 | 36 |
| N | 10 | 10 | 10 | 10 | 10 | 10 |

In the negative control group (G1), the tumor weight extracted after autopsy was shown as about 3.21g, but in the experimental groups, it was shown as 1.94, 2.17 and 1.95g, respectively, at the low, medium and high concentrations, and therefore the weight of the tumor tissue was shown significantly low. On the other hand, the total body weight in each group did not show a significant difference between the control group and experimental groups.

The tumor growth inhibition rate was 39.6% in the experimental group at the low concentration, and the tumor growth inhibition rate was 32.4% in the experimental group at the medium concentration, and the tumor growth inhibition rate was 39.3% in the experimental group at the high concentration, and therefore, it was confirmed that the tumor growth was inhibited 30% or more in all the experimental groups.

**8-3. Confirmation of necrosis and apoptosis of tumor cells**

Hematoxylin & Eosin staining to see necrosis of tumor cells in tumor tissue and TUNEL assay to see apoptosis were performed by conducting an autopsy after completing administration of strain CLCC1 for 36 days.

In FIG. 6e, a photograph of the H&E staining result for analysis of necrosis of tumor cells was shown. A significant difference was not observed in the level of necrosis of tumor cells between the negative control group and experimental groups. Thus, it can be found that *Faecalibacterium cancerinhibens* strain CLCC1 administered cell line of the present application does not induce an inflammatory response by necrosis of tumor.

In FIG. 6f, a photograph of the TUNEL staining analysis result for analysis of apoptosis of tumor cells was shown. It was confirmed that the apoptosis of tumor cells was statistically significantly increased in the strain CLCC1 administered groups, compared to the negative control group. Therefore, it was confirmed that the reduction of the tumor volume in each strain CLCC1 administered group was not caused by tumor necrosis, but by apoptosis of tumor, and did not induce an inflammatory response.

In Table 13 below, the result of comparing the necrosis and apoptosis of tumor cells was shown. In the table below, depending on the degree of apoptosis, ± represents minimal, and + represents mild, and ++ represents moderate, and +++ represents marked, and ++++ represents severe. Below each symbol, the number of killed cells corresponding to the corresponding stage is indicated.

[Table 13]

| Group Dose (cells head) | N | Apoptosis | | | | | | Necrosis | | | | |
|---|---|---|---|---|---|---|---|---|---|---|---|---|
| | | Rate(%) | ± | + | ++ | +++ | ++++ | ± | + | ++ | +++ | ++++ |
| G1/ 0 | 10 | 10.33=0.68 | 5 | 5 | 0 | 0 | 0 | 0 | 0 | 0 | 10 | 0 |
| G2 / $2\times10^6$ | 10 | 10.33=2.56 | 4 | 6 | 0 | 0 | 0 | 0 | 0 | 3 | 7 | 0 |
| G3 / $2\times10^7$ | 10 | 12.47=2.34## | 2 | 8 | 0 | 0 | 0 | 0 | 0 | 5 | 5 | 0 |
| G4 / $2\times10^8$ | 10 | 12.43±3.35 | 3 | 7 | 0 | 0 | 0 | 0 | 0 | 5 | 0 | 0 |
| ## p<0.01, Significant difference from the negative control group (G1) by Steel's t-test. | | | | | | | | | | | | |

As can be confirmed in the Table 13, the apoptosis ratio of about 10.33% was shown in the control group (Control, G1), and the apoptosis ratio similar to the control group was shown in the experimental group at the low concentration (G2), but the apoptosis of 12.4% or more was observed in the experimental groups at the medium concentration (G3) and high concentration (G4). Accordingly, it was confirmed that the apoptosis of tumor cells was increased by administration of the *Faecalibacterium cancerinhibens* strain CLCC1.

**Example 9: Evaluation of effect of *Faecalibacterium* spp. microorganism using colorectal cancer subcutaneous tumor model**

(1) Animal model preparation

To confirm the anti-cancer effect against colorectal cancer during administration of strain CLCC1, mice in which a colorectal cancer cell was subcutaneously transplanted were used, and a test was performed in Champion's oncology, a non-clinical commission testing institution in the United States. As the colorectal cancer cell, a mouse-derived colorectal cancer cell line, MC38 cell was used, and as the mouse strain, C57BL/6 was used. The MC38 cell is a cancer cell line corresponding mouse-derived colorectal carcinoma. The test group is as follows, and 10 mice were included per group.

Specifically, through a subcutaneous tumor transplantation experiment of MC38 cell line, the colorectal cancer cell line, in 6-week-old male C57BL/6 mice, colorectal cancer was caused, and the effect of the oral administration of *Faecalibacterium cancerinhibens* strain CLCC1 on the growth of colorectal cancer cells during the process of colorectal cancer occurrence was examined.

(2) Preparation of test formulations

By the substantially same method as the preparation method of test formulations of the control group and experimental groups of Example 8-1, *Faecalibacterium cancerinhibens* strain CLCC1 of Example 1 was cultured in an RCM medium, and then concentrated and stored frozen to final glycerol concentration of 15%(v/v) using PBS buffer and glycerol. In addition, after hawing immediately before administration to mice, low concentration living cells at a content of 200ul per animal ($2\times10^7$ cells/dose) (Sample 1 corresponds to G3 of Example 8-1) and high concentration living cells ($2\times10^8$ cells/dose) at a content of 200ul per animal ($2\times10^8$cells) (Sample 2 corresponds to G4 of Example 8-1) were orally administered. As a control group, 10 mice not administered with strain CLCC1 were used.

Sample 3 was used as high concentration dead cells ($2\times10^8$ cells/dose) by treating the prepared Sample 2 in an oven at a temperature of 100°C for 2 hours.

(3) Effect evaluation

As shown in the test design of FIG. 8, the test formulations of the control group and Samples 1 to 3 were administered once a day for 40 days in total by starting administration 2 weeks before inoculating cancer cells. Specifically, the test formulations of the control group and Samples 1 to 3 were orally administered once a day for 40 days, and after 14 days from the start date of administration, colorectal cancer cells ($5\times10^5$ MC38 cells in 0.1ml PBS) were inoculated to the subcutaneous tissue of the left flank, and after a one-week engraftment period, the tumor size was observed from the 22$^{nd}$ day to the 40$^{th}$ day for 18 days. Day 22, the start date of tumor observation was regarded as Day 0, and the size of the colorectal cancer tumor (tumor volume) was measured using Magnetic Resonance (MR) imaging for 18 days. The change in the tumor size was calculated by measuring the maximum length (L) and perpendicular width (W) of tumor for 22 days after engraftment of cancer cells and introducing them to the following Equation 3. The tumor volume measured for 18 days was shown in Table 13 and FIG. 7 below.

[Equation 3]

$$\text{Tumor volume (TV) (mm}^3\text{)} = \text{L (mm)} \times \text{W}^2\ \text{(mm}^2\text{)} \times 1/2$$

In the equation, L means the maximum length (L) of tumor and W2 means perpendicular width (W). The relative tumor volume of the sample based on the tumor volume of the control group was calculated by Equation 2 below.

[Equation 2]

$$\text{Relative reduction rate of tumor volume (\%)} = (1-(T2/C2))*100$$

In Table 13 below, Mean means the average tumor volume and S.D. means standard deviation. Table 13 is the result of the tumor volume change in the colorectal cancer cell linetransplanted subcutaneous tumor model in which the isolated strain according to the control group and Samples 1 to 3 was administered.

[Table 14] Tumor volume ($mm^3$)

| Observation date | **Control group** | | **Sample 1** | | **Sample 2** | | **Sample 3** | |
|---|---|---|---|---|---|---|---|---|
| | Mean | S.D. | Mean | S.D. | Mean | S.D. | Mean | S.D. |
| **1** | 28.63 | 19.64 | 18.3 | 13.12 | 29.1 | 18.21 | 19 | 14.64 |
| **4** | 47.63 | 27.39 | 44.8 | 33.41 | 60.7 | 89.62 | 42.3 | 42.41 |
| **7** | 109.38 | 60.98 | 92.6 | 59.67 | 100.6 | 111.84 | 85.5 | 89.91 |
| **8** | 174.63 | 33.93 | 171.7 | 124.72 | 231.4 | 319.62 | 124.2 | 97.38 |
| **11** | 367.63 | 83.97 | 295.2 | 201.81 | 303.8 | 360.13 | 277.2 | 265.91 |
| **13** | 633.75 | 218.75 | 437.4 | 307.75 | 446.1 | 457.75 | 396.8 | 388.22 |
| **15** | 934.13 | 329.64 | 686.6 | 495.19 | 785.5 | 694.4 | 707.4 | 679.62 |
| **18** | 1441.86 | 444.79 | 1296 | 979.09 | 1070.56 | 852.65 | 1094.89 | 897 |

As the result, the tumor volume change obtained in the groups administered with the strain of Samples 1 to 3 compared to the control group was shown in FIG. 7, respectively. Based on the last day of measurement, as the result of calculating the tumor volume of Sample 1 to Sample 3 by percentage by setting the tumor volume of the control group to 100%, Sample 1 was 89.9% (10.1% reduction), and Sample 2 was 74.2% (25.8% reduction), and Sample 3 was 75.9% (24.1 reduction), and therefore, as the test result, it was confirmed that the tumor growth was statistically significantly inhibited in the colorectal cancer model mice administered with strain CLCC1, compared to the control group.

It was confirmed that the tumor growth was statistically significantly inhibited even when dead cells of strain CLCC1 were administered. In addition, regarding the reduction of the tumor volume of Sample 1 and Sample 2 using living cells of strain CLCC1 strain but at different concentrations, it was confirmed that the anti-cancer activity was increased in a concentration-dependent manner under the same experimental condition.

In addition, after the day of administrating the test formulations, at an interval of 1 to 3 days, general symptoms such as appearance, body weight, behavior, excretion, and the like were observed. The test was performed for 40 days in total after administration of the test formulations, and it was observed for 18 days after engraftment of cancer cells. The measured body weight (Kg) of the experimental animals was shown in Table 15 below.

[Table 15]

| Observation date | Control group | Sample 1 | Sample 2 | Sample 3 |
|---|---|---|---|---|
| 1 | 20.60 | 20.79 | 20.60 | 21.17 |
| 4 | 20.03 | 20.62 | 21.31 | 21.64 |
| 7 | 20.24 | 20.47 | 21.42 | 21.55 |
| 8 | 20.26 | 20.41 | 21.22 | 21.52 |
| 11 | 20.05 | 20.44 | 21.06 | 21.22 |
| 13 | 20.42 | 20.44 | 21.17 | 21.28 |
| 15 | 20.54 | 20.77 | 21.13 | 21.38 |
| 18 | 20.50 | 20.93 | 21.27 | 21.09 |

For the total body weight of the experimental animals, the body weight of mice corresponding to the control group tended to be lower than that of the test groups after engraftment of cancer cells, but there was no significant difference between the control group and the test groups overall.

## Claims

1. A *Faecalibacterium* spp. microorganism comprising a 16S rRNA gene comprising a nucleotide sequence having 97% or more sequence identity with a nucleotide sequence of SEQ ID NO: 1.

2. The *Faecalibacterium* spp. microorganism according to claim 1, wherein the microorganism comprises one or more fatty acids selected from the group consisting of 15:1 ω8*c* fatty acid, 19:0 cyclo ω10*c*/19ω6 fatty acid, 20:1 ω9*c* fatty acid, 14:0 3OH/16:1 iso I fatty acid, 15:0 3OH fatty acid, 16:0 iso fatty acid and 16:0 iso 3OH fatty acid.

3. The *Faecalibacterium* spp. microorganism according to claim 1, wherein the microorganism does not comprise 17:0 iso 3OH fatty acid.

4. The *Faecalibacterium* spp. microorganism according to claim 1, wherein the microorganism has an anti-cancer activity to induce a tumor growth inhibitory activity or apoptosis of cancer cells.

5. The *Faecalibacterium* spp. microorganism according to claim 1, wherein the relative reduction rate of the tumor volume is 5% or more, when the microorganism is administered to an animal model transplanted with cancer cell lines, compared to a control group not being administered by the microorganism.

6. The *Faecalibacterium* spp. microorganism according to claim 1, wherein the inhibition rate of the tumor growth according to the tumor weight is 5% or more, when the microorganism is administered to an animal model transplanted with cancer cell lines, compared to a control group not being administered by the microorganism.

7. The *Faecalibacterium* spp. microorganism according to claim 1, wherein the microorganism is cultured at a temperature of 25 to 40 °C under an anaerobic condition.

8. The *Faecalibacterium* spp. microorganism according to claim 1, wherein the microorganism is *Faecalibacterium cancerinhibens.*

9. The *Faecalibacterium* spp. microorganism according to claim 8, wherein the microorganism is *Faecalibacterium cancerinhibens* strain CLCC1 deposited with accession number KCTC 13783BP.

10. An anti-cancer composition, comprising at least an active ingredient selected from the group consisting of a microbial cell of the *Faecalibacterium* spp. microorganism according to any one of claim 1 to claim 9, a culture of the microorganism, a lysate of the microorganism and an extract of the microbial cell, culture or lysate.

11. The composition according to claim 10, wherein the composition induces a tumor growth inhibitory activity or apoptosis of cancer cells.

12. The composition according to claim 10, wherein the cancer is esophageal cancer, gallbladder cancer, liver cancer, biliary tract cancer, pancreatic cancer, stomach cancer, small intestine cancer, colorectal cancer, colon cancer, anal cancer, rectal cancer, leukemia, prostate cancer, breast cancer, bladder cancer, kidney cancer, multiple myeloma, cervical cancer, thyroid cancer, ovarian cancer, urethral cancer, osteosarcoma, glioblastoma, brain tumor or lymphoma.

13. The composition according to claim 10, wherein the cancer is one or more selected from the group consisting of colorectal cancer and liver cancer.

14. The composition according to claim 10, wherein the anti-cancer composition is a pharmaceutical composition or food composition.

**15.** The composition according to claim 10, wherein the composition is a probiotic.

**16.** The composition according to claim 15, wherein the composition comprises a probiotic and a prebiotic.

【FIG. 1a】

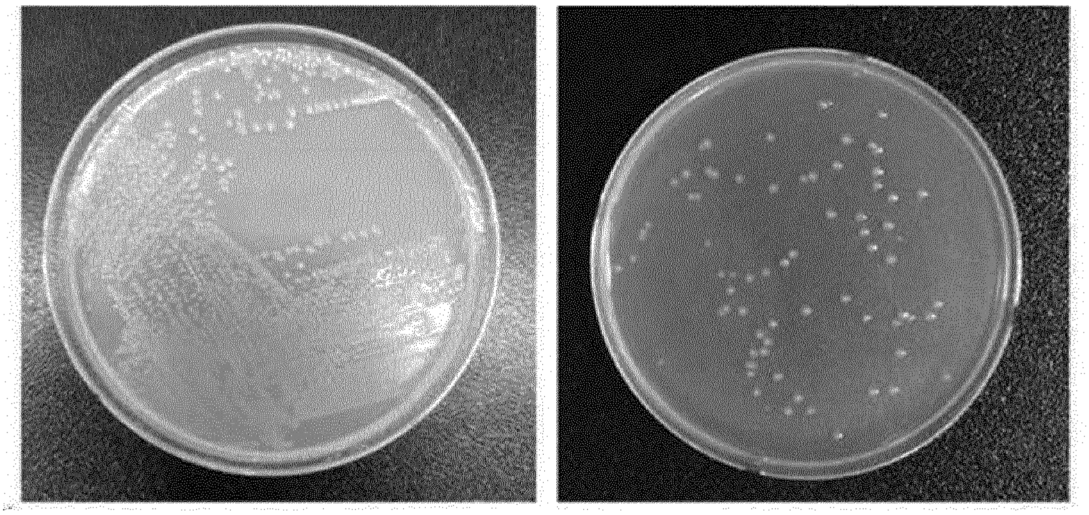

【FIG. 1b】

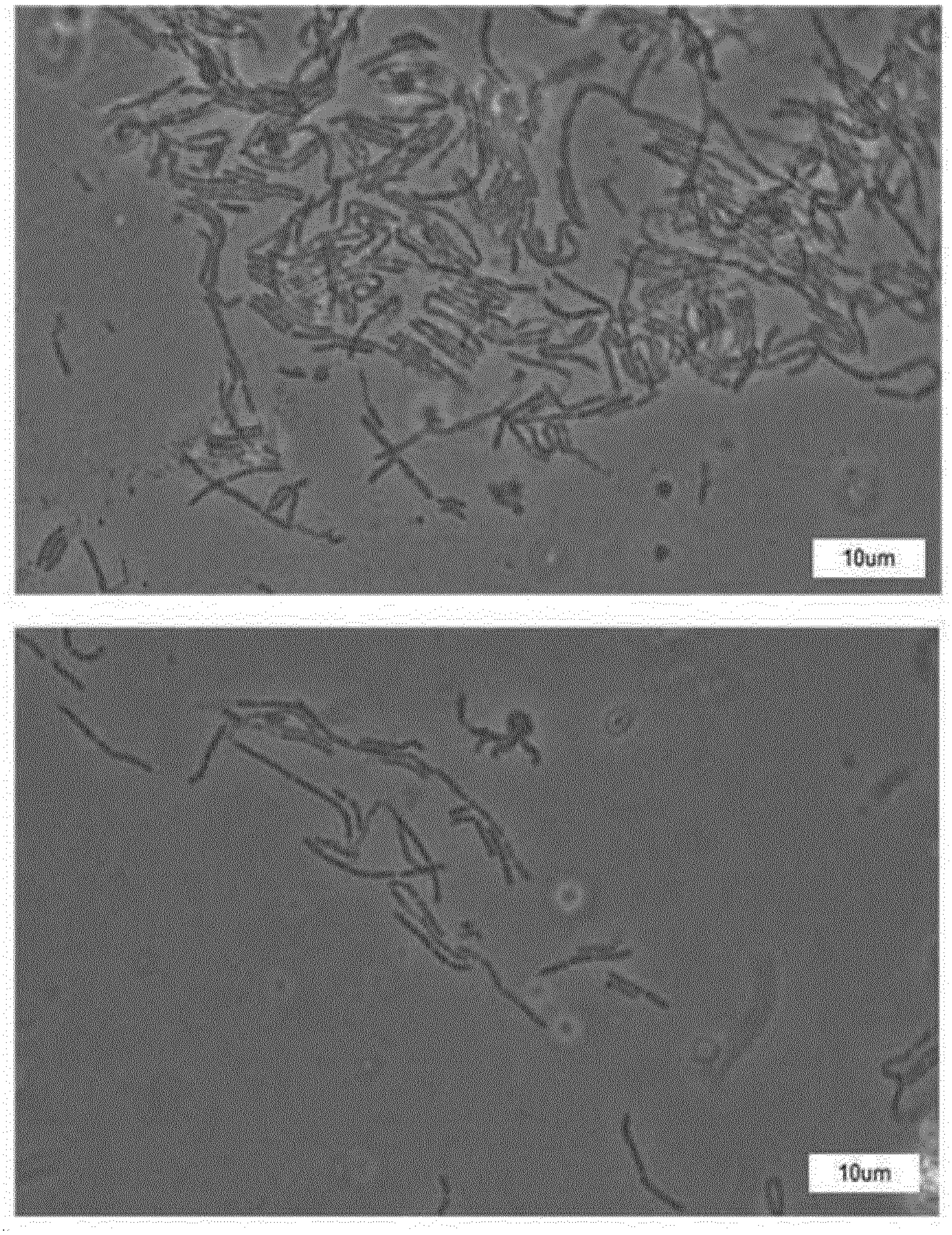

【FIG. 1c】

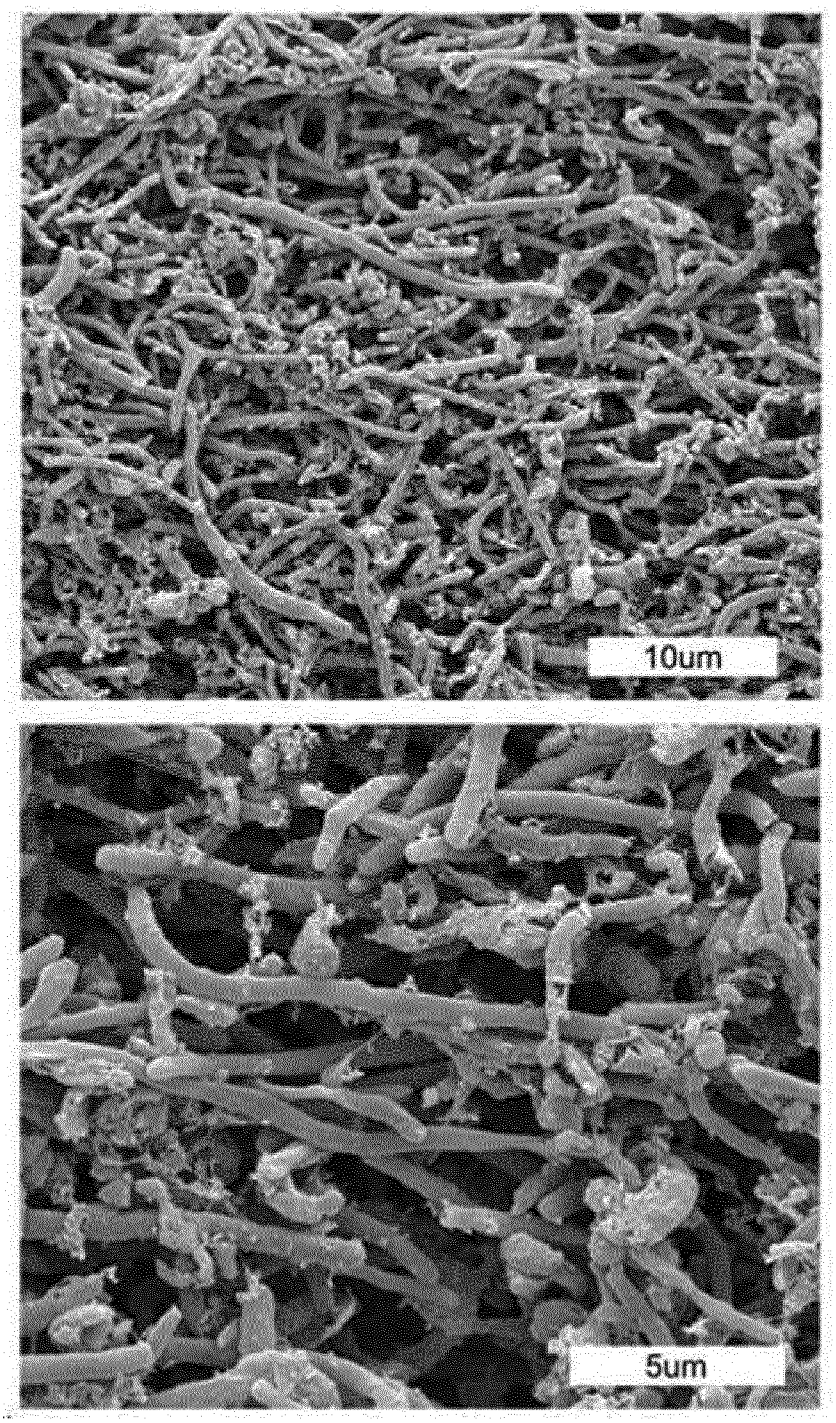

【FIG. 2a】

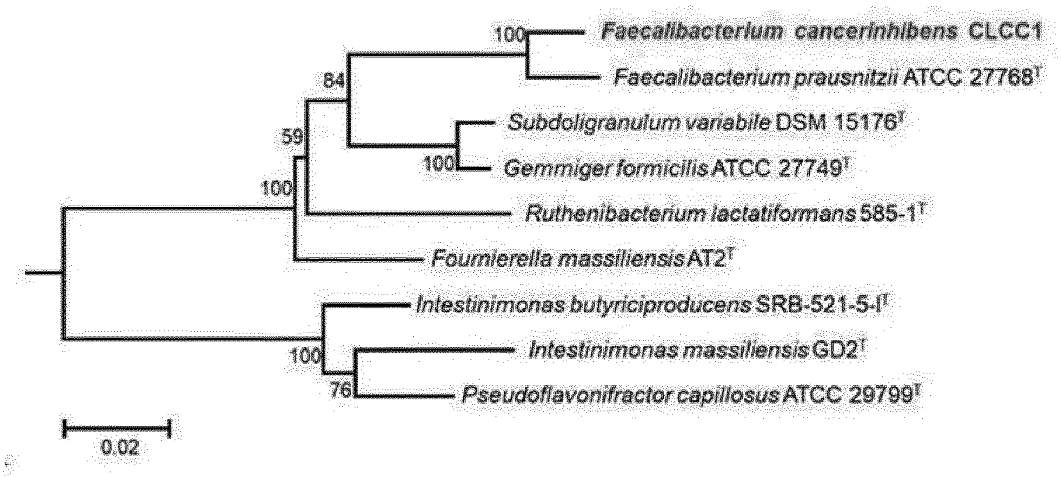
100
Faecalibacterium cancerinhibens CLCC1
Faecalibacterium prausnitzii ATCC 27768T
84
Subdoligranulum variabile DSM 15176T
59
100
Gemmiger formicilis ATCC 27749T
100
Ruthenibacterium lactatiformans 585-1T
Fournierella massiliensis AT2T
Intestinimonas butyriciproducens SRB-521-5-IT
100
Intestinimonas massiliensis GD2T
76
Pseudoflavonifractor capillosus ATCC 29799T
0.02

【FIG. 2b】

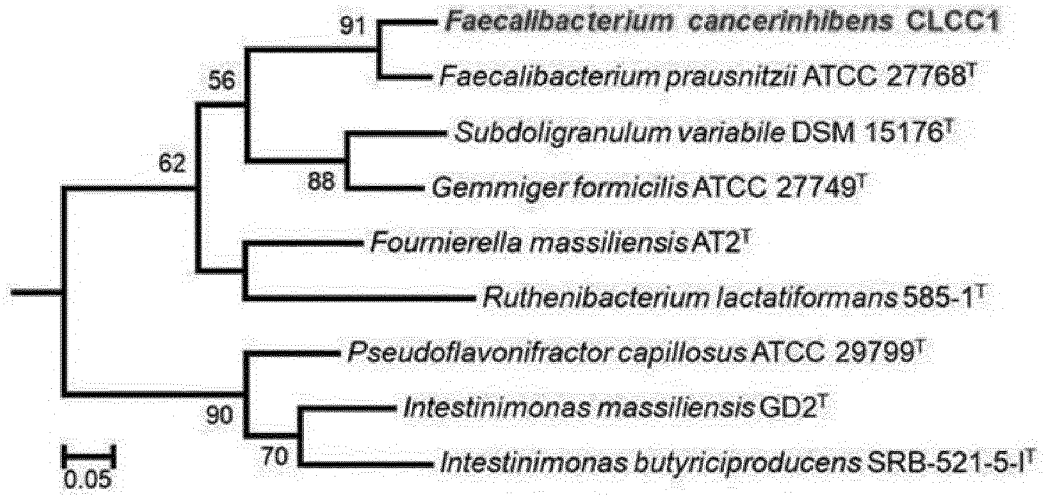
91
Faecalibacterium cancerinhibens CLCC1
56
Faecalibacterium prausnitzii ATCC 27768T
Subdoligranulum variabile DSM 15176T
62
88
Gemmiger formicilis ATCC 27749T
Fournierella massiliensis AT2T
Ruthenibacterium lactatiformans 585-1T
Pseudoflavonifractor capillosus ATCC 29799T
90
Intestinimonas massiliensis GD2T
70
Intestinimonas butyriciproducens SRB-521-5-IT
0.05

【FIG. 2c】

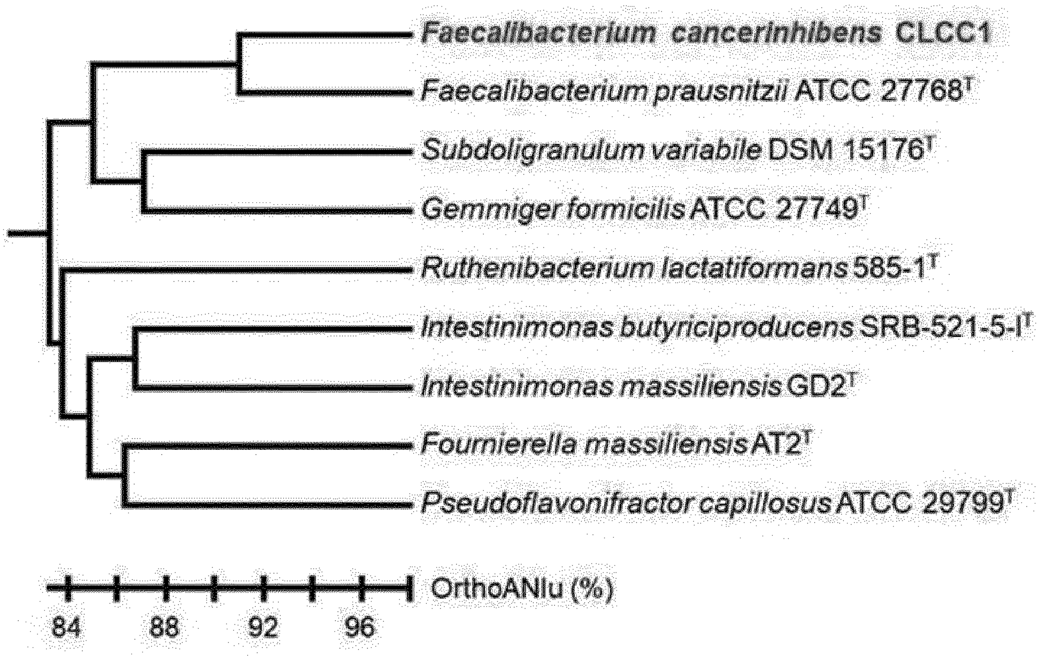
Faecalibacterium cancerinhibens CLCC1
Faecalibacterium prausnitzii ATCC 27768T
Subdoligranulum variabile DSM 15176T
Gemmiger formicilis ATCC 27749T
Ruthenibacterium lactatiformans 585-1T
Intestinimonas butyriciproducens SRB-521-5-IT
Intestinimonas massiliensis GD2T
Fournierella massiliensis AT2T
Pseudoflavonifractor capillosus ATCC 29799T
OrthoANIu (%)
84
88
92
96

【FIG. 2d】

## comparison of average nucleotide indentity

| | 1 | 2 | 3 | 4 | 5 | 6 | 7 | 8 | 9 |
|---|---|---|---|---|---|---|---|---|---|
| 1 | 100.00 | 85.99 | 71.86 | 67.41 | 68.78 | 69.34 | 70.49 | 72.73 | 74.50 |
| 2 | 85.99 | 100.00 | 72.49 | 69.06 | 69.22 | 70.58 | 71.34 | 72.94 | 75.81 |
| 3 | 71.86 | 72.49 | 100.00 | 69.43 | 73.69 | 76.62 | 74.94 | 73.44 | 72.01 |
| 4 | 67.41 | 69.06 | 69.43 | 100.00 | 77.58 | 74.62 | 67.10 | 68.92 | 67.74 |
| 5 | 68.78 | 69.22 | 73.69 | 77.58 | 100.00 | 77.52 | 71.59 | 72.95 | 68.57 |
| 6 | 69.34 | 70.58 | 76.62 | 74.62 | 77.52 | 100.00 | 72.66 | 74.07 | 69.39 |
| 7 | 70.49 | 71.34 | 74.94 | 67.10 | 71.59 | 72.66 | 100.00 | 71.81 | 70.68 |
| 8 | 72.73 | 72.94 | 73.44 | 68.92 | 72.95 | 74.07 | 71.81 | 100.00 | 78.17 |
| 9 | 74.50 | 75.81 | 72.01 | 67.74 | 68.57 | 69.39 | 70.68 | 78.17 | 100.00 |

1, CLCC1; 2, *Faecalibacterium prausnitzii* ATCC 27768(T); 3, *Fournierella massiliensis* AT2(T); 4, *Intestinimonas butyriciproducens* DSM 26588(T); 5, *Intestinimonas massiliensis* GD2(T); 6, *Pseudoflavonifractor capillosus* ATCC 29799(T); 7, *Ruthenibacterium lactatiformans* 585-1(T); 8, *Subdoligranulum variabile* DSM 15176(T); 9, *Gemmiger formicilis* ATCC 27749(T)

【FIG. 3a】

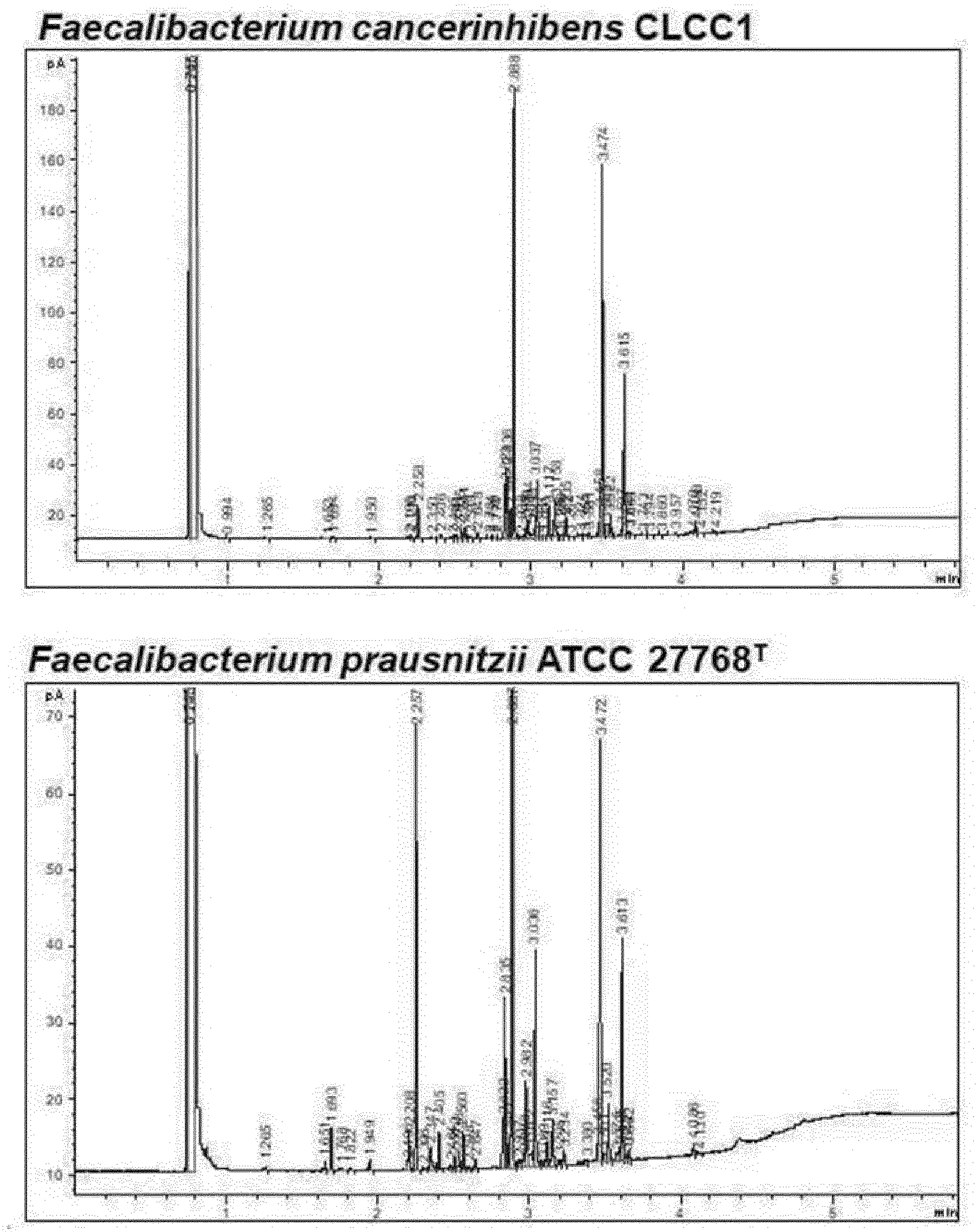
Faecalibacterium cancerinhibens CLCC1
pA
min
Faecalibacterium prausnitzii ATCC 27768T
pA
min

【FIG. 3b】

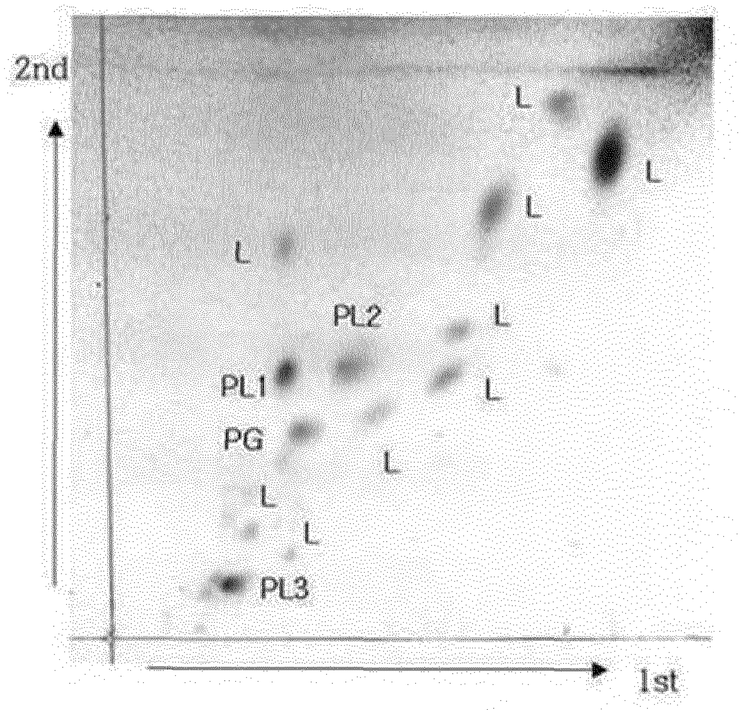


**(B)** ***Faecalibacterium prausnitzii*** **ATCC 27768[T]**

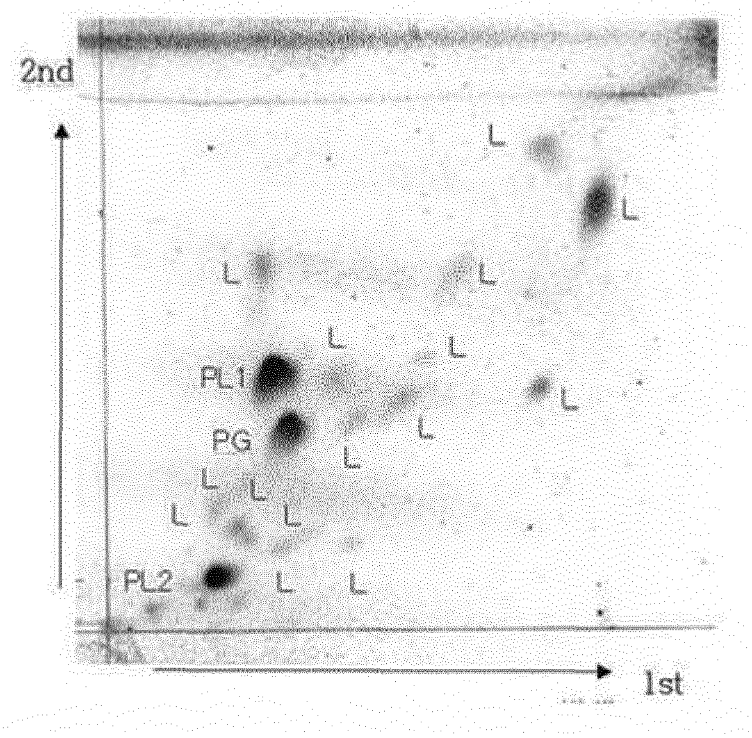

【FIG. 3c】

*Faecalibacterium cancerinhibens* CLCC1

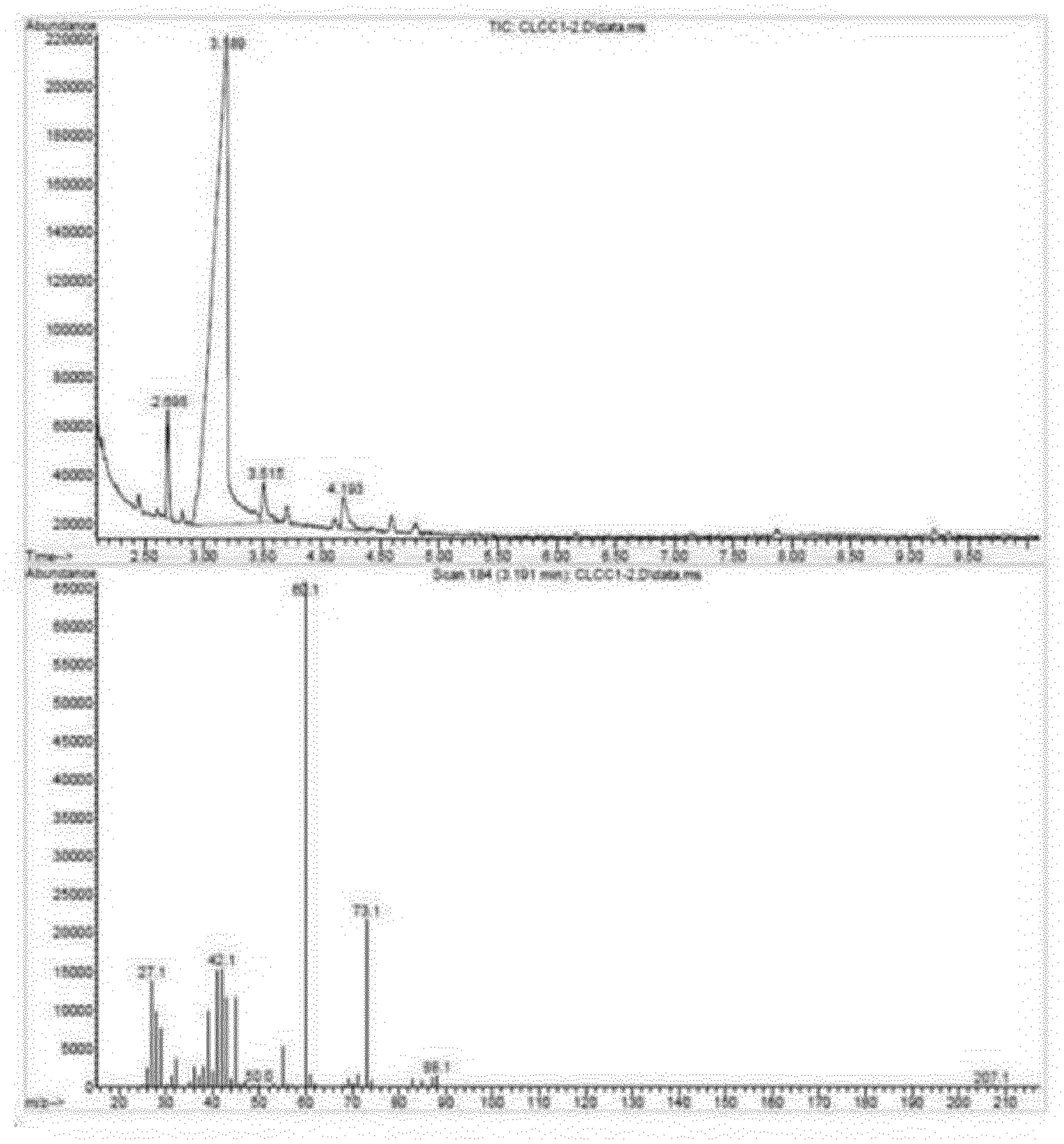

【FIG. 3d】

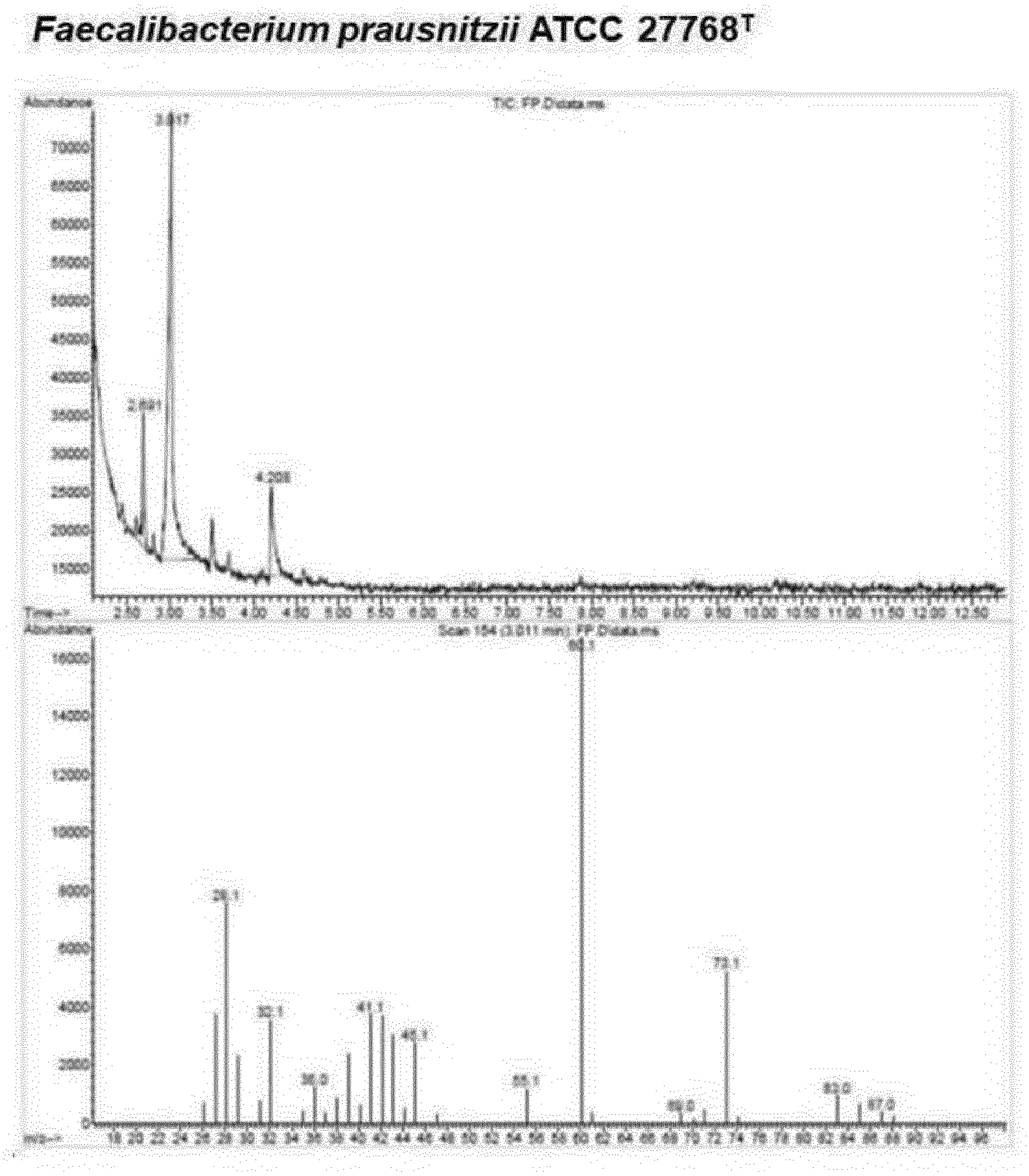
Faecalibacterium prausnitzii ATCC 27768T
Abundance
TIC: FP D\data.ms
3.017
2.691
4.208
Time-->
Abundance
60.1
28.1
32.1
41.1
45.1
36.0
55.1
73.1
69.0
83.0
87.0
m/z-->

【FIG. 4a】

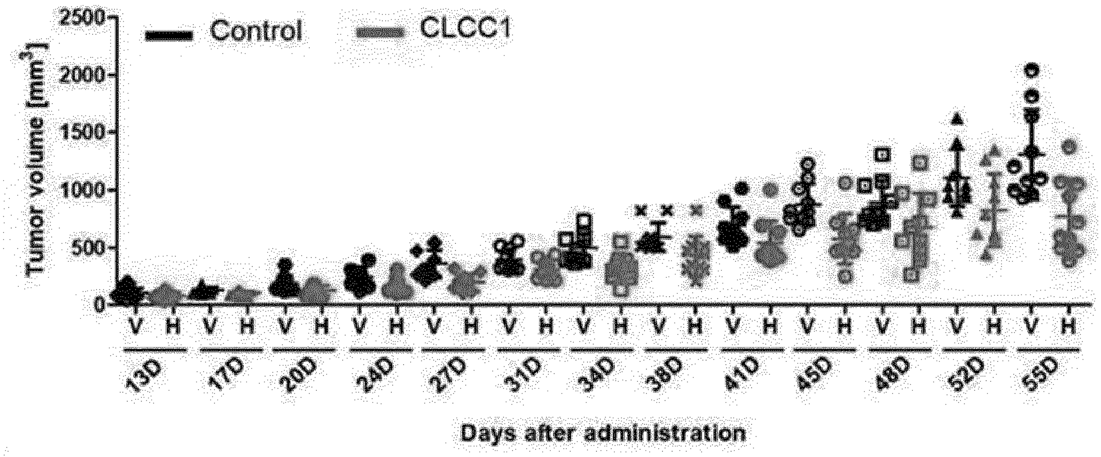
Control
CLCC1
Tumor volume [mm3]
2500
2000
1500
1000
500
0
V H V H V H V H V H V H V H V H V H V H V H V H V H
13D 17D 20D 24D 27D 31D 34D 38D 41D 45D 48D 52D 55D
Days after administration

【FIG. 4b】

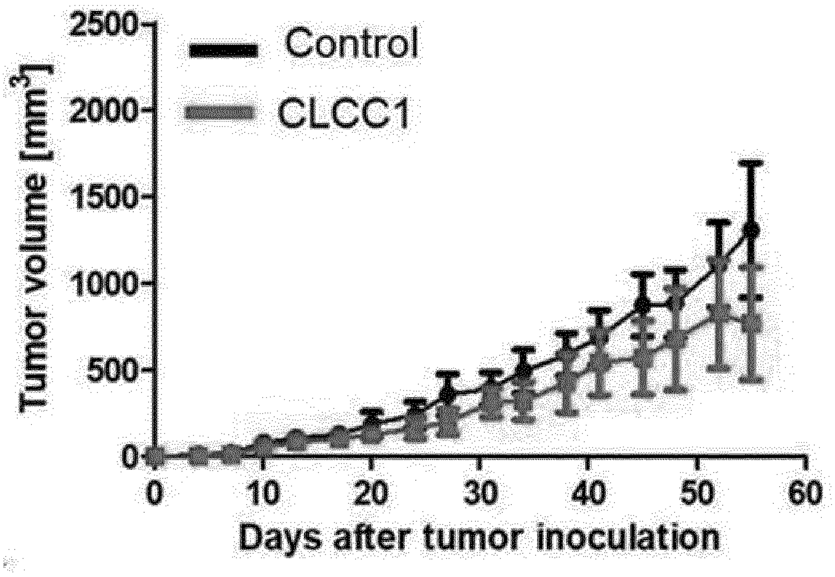
Control
CLCC1
Tumor volume [mm3]
2500
2000
1500
1000
500
0
0 10 20 30 40 50 60
Days after tumor inoculation

【FIG. 4c】

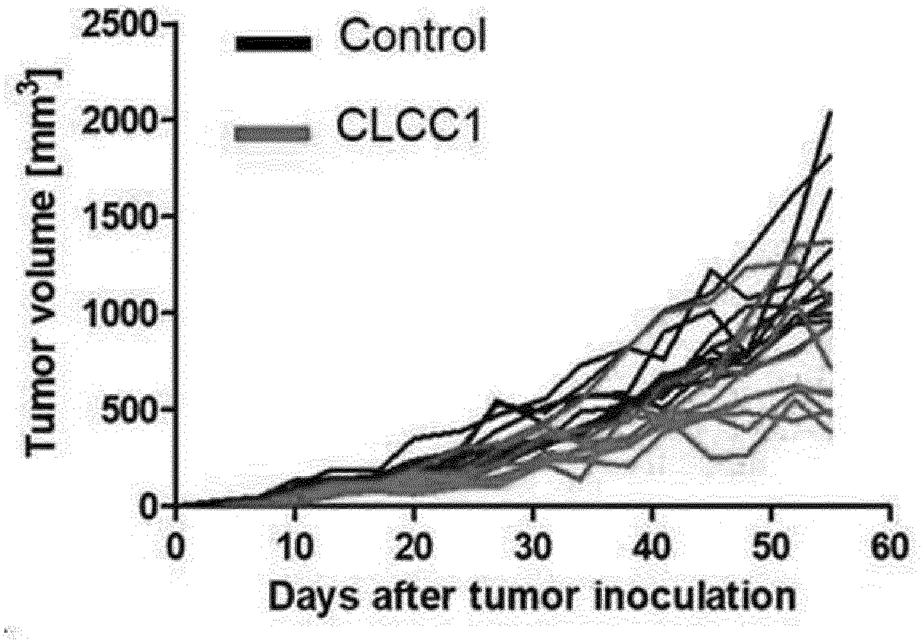
Control
CLCC1
Tumor volume [mm3]
2500
2000
1500
1000
500
0
0
10
20
30
40
50
60
Days after tumor inoculation

【FIG. 5a】

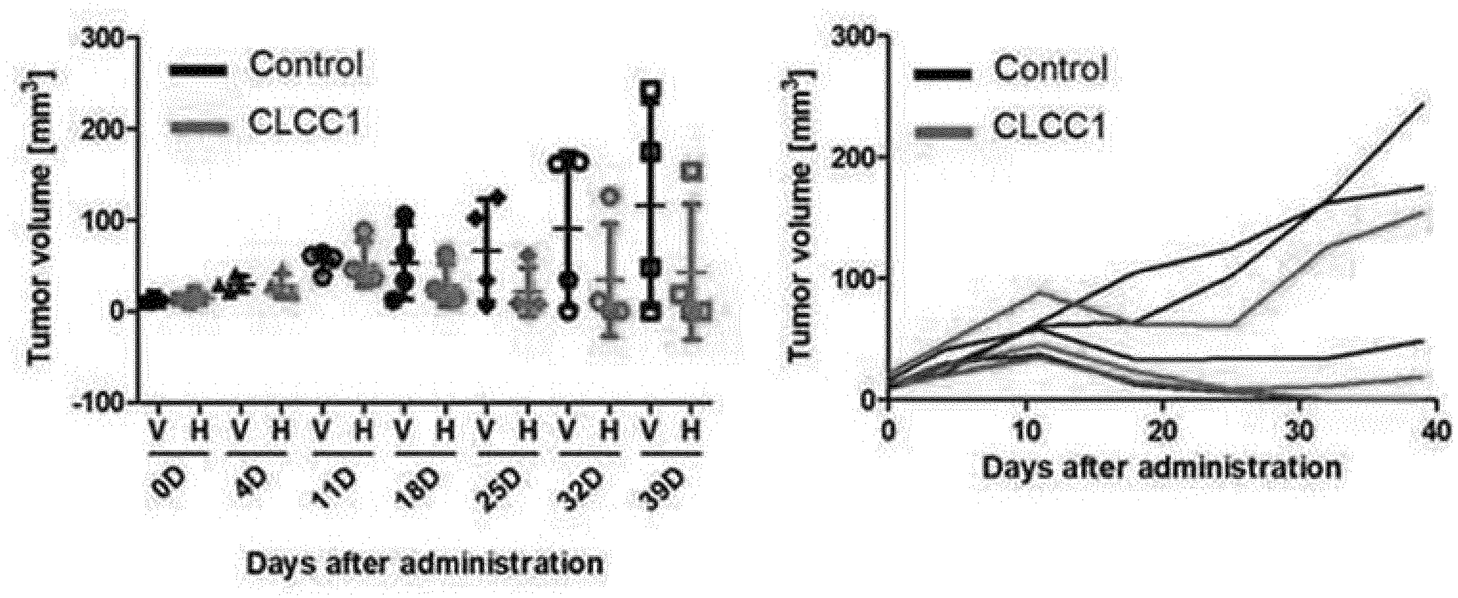


| | Control | | | | CLCC1 | | | |
|---|---|---|---|---|---|---|---|---|
| Days after administration | 1 | 2 | 3 | 4 | 1 | 2 | 3 | 4 |
| 0 | 11.1 | 12.1 | 15.9 | 9.5 | 9.4 | 21.1 | 13.7 | 12.9 |
| 4 | 21.7 | 29.5 | 41.4 | 26.8 | 20.2 | 46.1 | 19.5 | 27.7 |
| 11 | 63.8 | 37.9 | 58.4 | 61.1 | 36.5 | 88.2 | 34.7 | 45.7 |
| 18 | 105.2 | 12.2 | 33.0 | 64.1 | 14.6 | 62.5 | 14.2 | 23.1 |
| 25 | 124.8 | 6.1 | 33.8 | 101.9 | 8.6 | 61.0 | 7.1 | 8.4 |
| 32 | 162.3 | 0.0 | 34.3 | 164.1 | 11.0 | 126.0 | 0.0 | 0.0 |
| 39 | 174.8 | 0.0 | 48.5 | 243.0 | 18.7 | 154.2 | 0.0 | 0.0 |

【FIG. 5b】

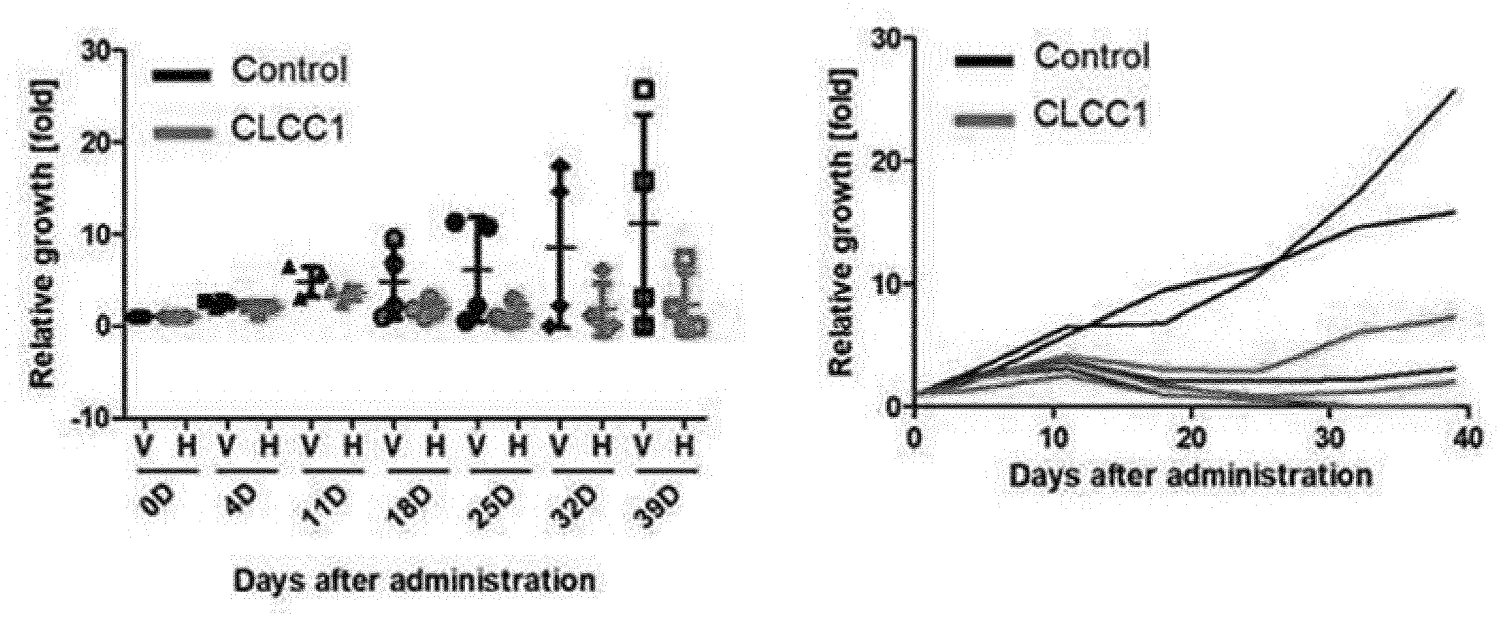


| Days after administration | Control | | | | CLCC1 | | | |
|---|---|---|---|---|---|---|---|---|
| | 1 | 2 | 3 | 4 | 1 | 2 | 3 | 4 |
| 0 | 1.0 | 1.0 | 1.0 | 1.0 | 1.0 | 1.0 | 1.0 | 1.0 |
| 4 | 2.0 | 2.4 | 2.6 | 2.8 | 2.2 | 2.2 | 1.4 | 2.2 |
| 11 | 5.8 | 3.1 | 3.7 | 6.5 | 3.9 | 4.2 | 2.5 | 3.6 |
| 18 | 9.5 | 1.0 | 2.1 | 6.8 | 1.6 | 3.0 | 1.0 | 1.8 |
| 25 | 11.3 | 0.5 | 2.1 | 10.8 | 0.9 | 2.9 | 0.5 | 0.7 |
| 32 | 14.6 | 0.0 | 2.2 | 17.4 | 1.2 | 6.0 | 0.0 | 0.0 |
| 39 | 15.8 | 0.0 | 3.1 | 25.7 | 2.0 | 7.3 | 0.0 | 0.0 |

【FIG. 5c】

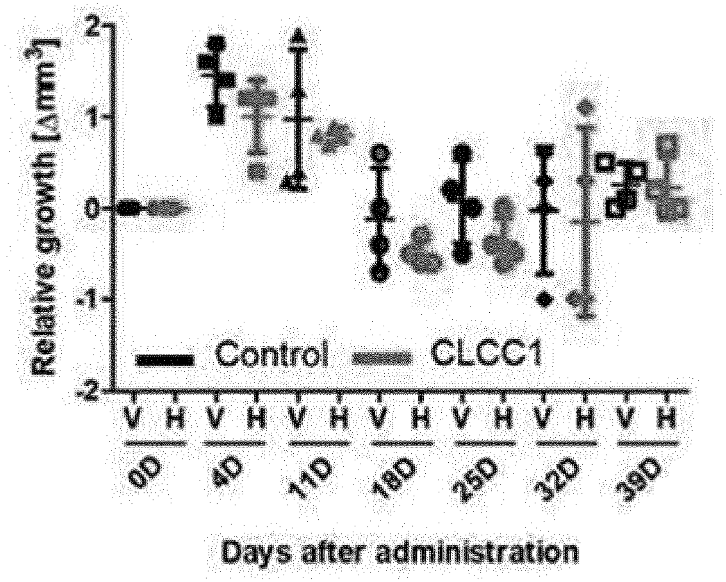


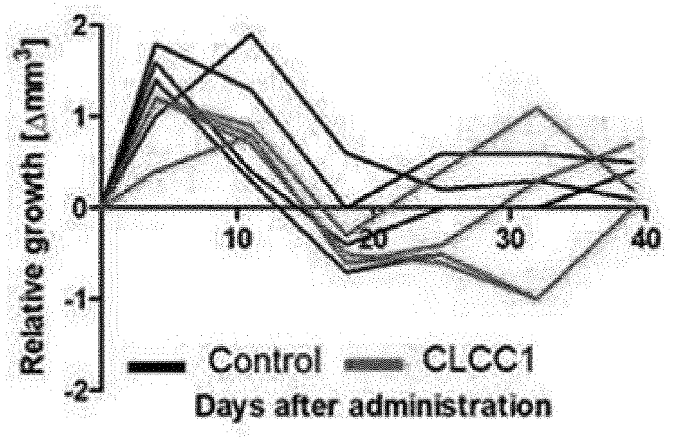


| | Control | | | | CLCC1 | | | |
|---|---|---|---|---|---|---|---|---|
| Days after administration | 1 | 2 | 3 | 4 | 1 | 2 | 3 | 4 |
| 0 | 0 | 0.0 | 0.0 | 0.0 | 0.0 | 0.0 | 0.0 | 0.0 |
| 4 | 1.0 | 1.4 | 1.6 | 1.8 | 1.2 | 1.2 | 0.4 | 1.2 |
| 11 | 1.9 | 0.3 | 0.4 | 1.3 | 0.8 | 0.9 | 0.8 | 0.7 |
| 18 | 0.6 | -0.7 | -0.4 | 0.0 | -0.6 | -0.3 | -0.6 | -0.5 |
| 25 | 0.2 | -0.5 | 0.0 | 0.6 | -0.4 | -0.0 | -0.5 | -0.6 |
| 32 | 0.3 | -1.0 | 0.0 | 0.6 | 0.3 | 1.1 | -1.0 | -1.0 |
| 39 | 0.1 | 0.0 | 0.4 | 0.5 | 0.7 | 0.2 | 0.0 | 0.0 |

【FIG. 6a】

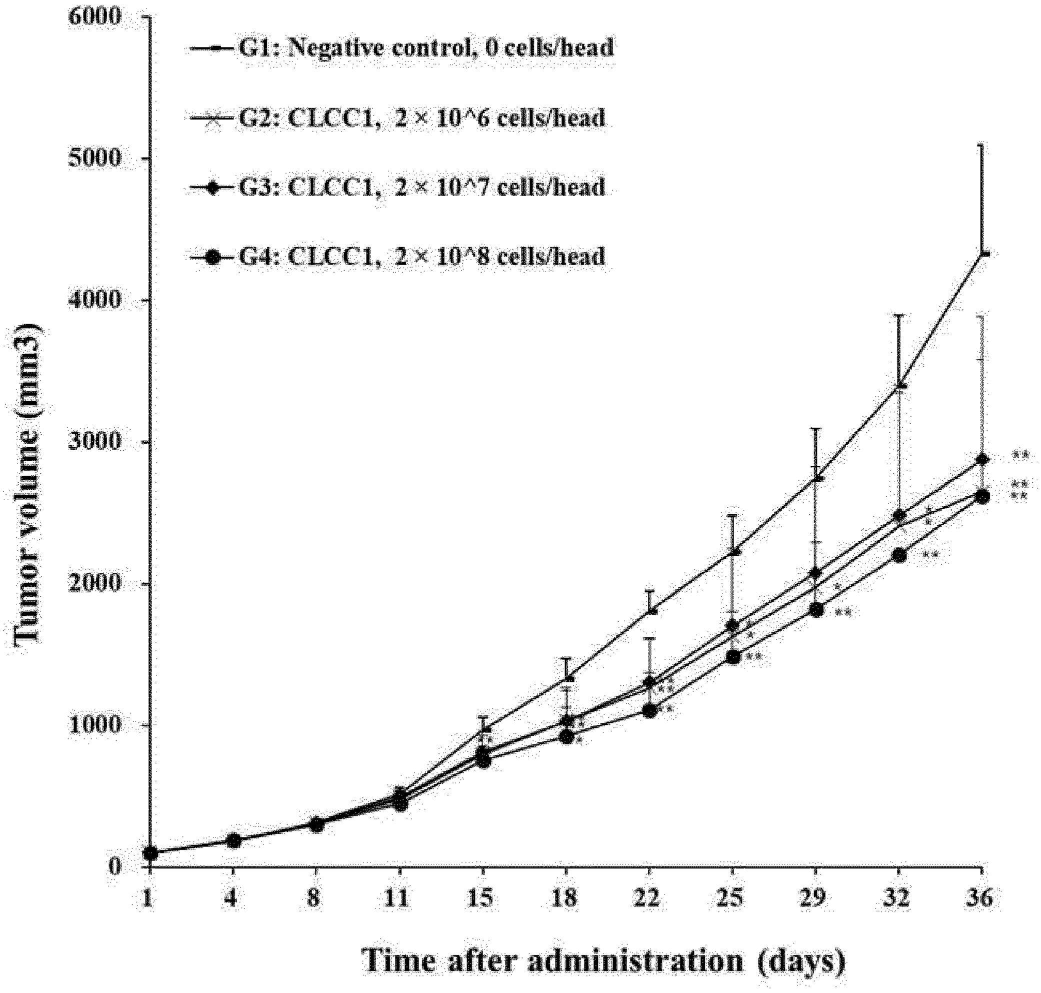


Each point represents the mean + S.D. (n=10)
* p<0.05, Significant difference from the negative control group (G1) by Dunnett's t-test.
** p<0.01, Significant difference from the negative control group (G1) by Dunnett's t-test.

【FIG. 6b】

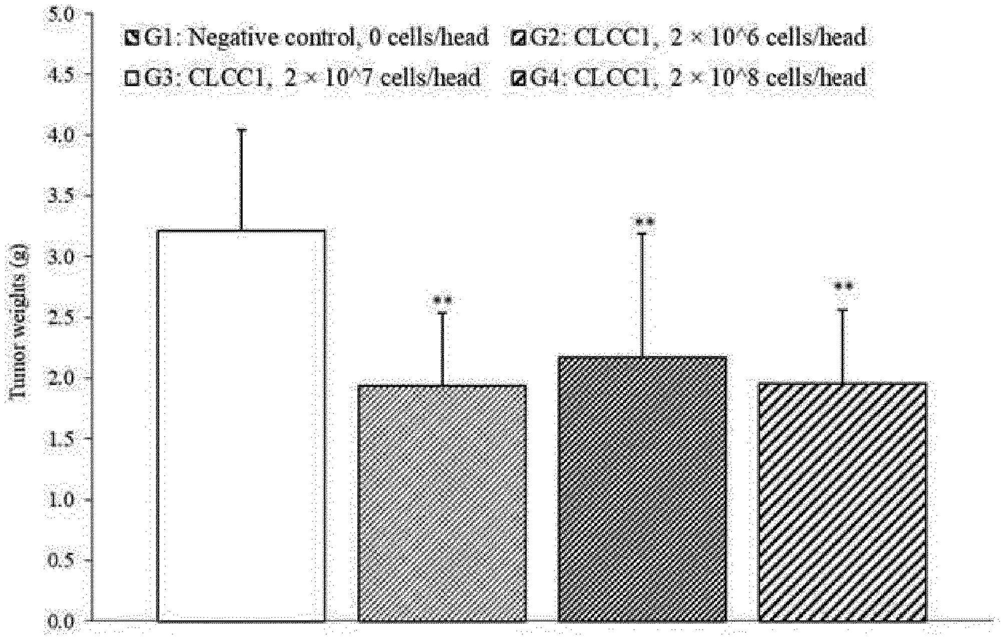


Each point represents the mean + S.D. (n=10)
** p<0.01, Significant difference from the negative control group (G1) by Dunnett's t-test.

【FIG. 6c】

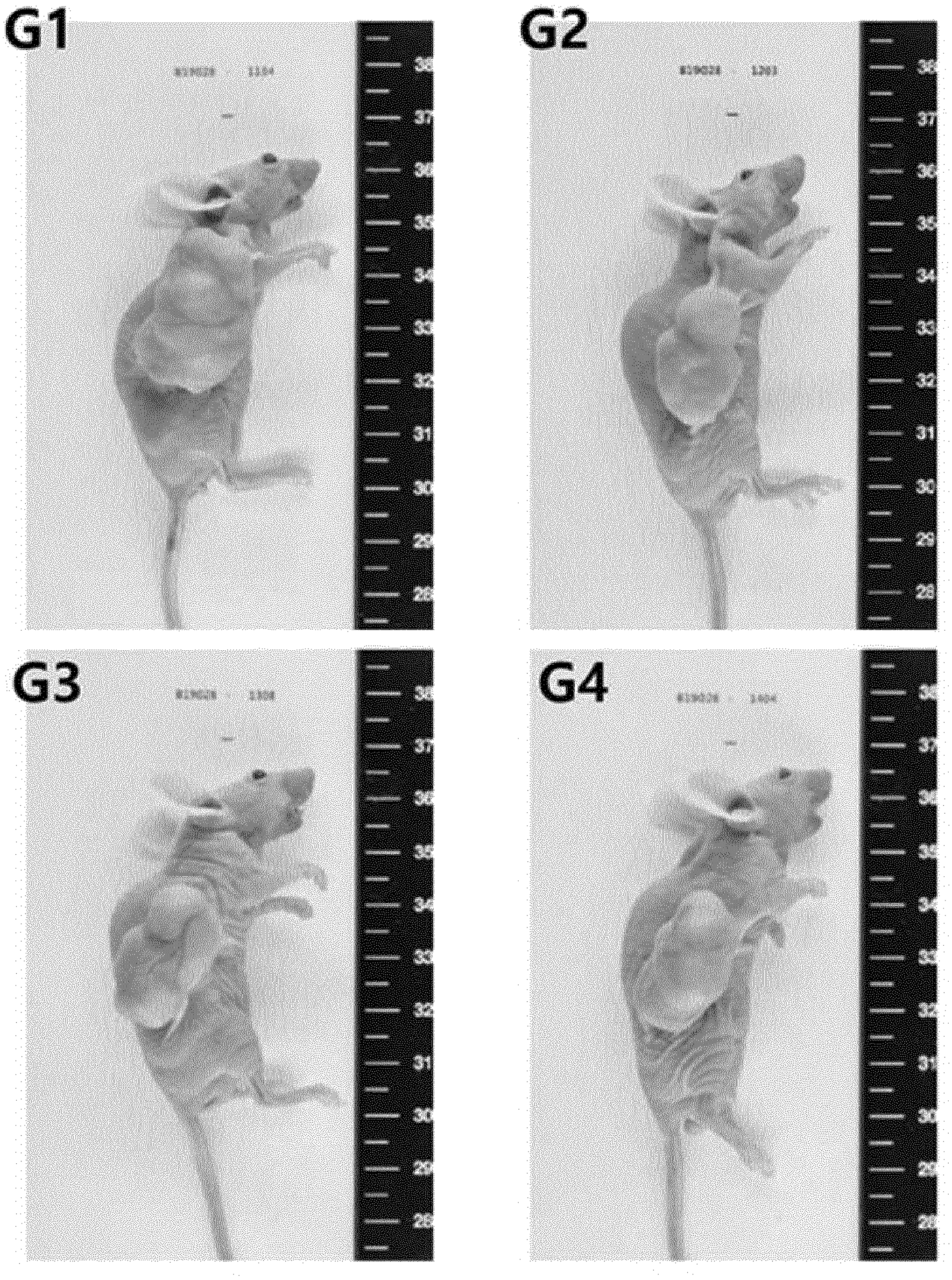

【FIG. 6d】

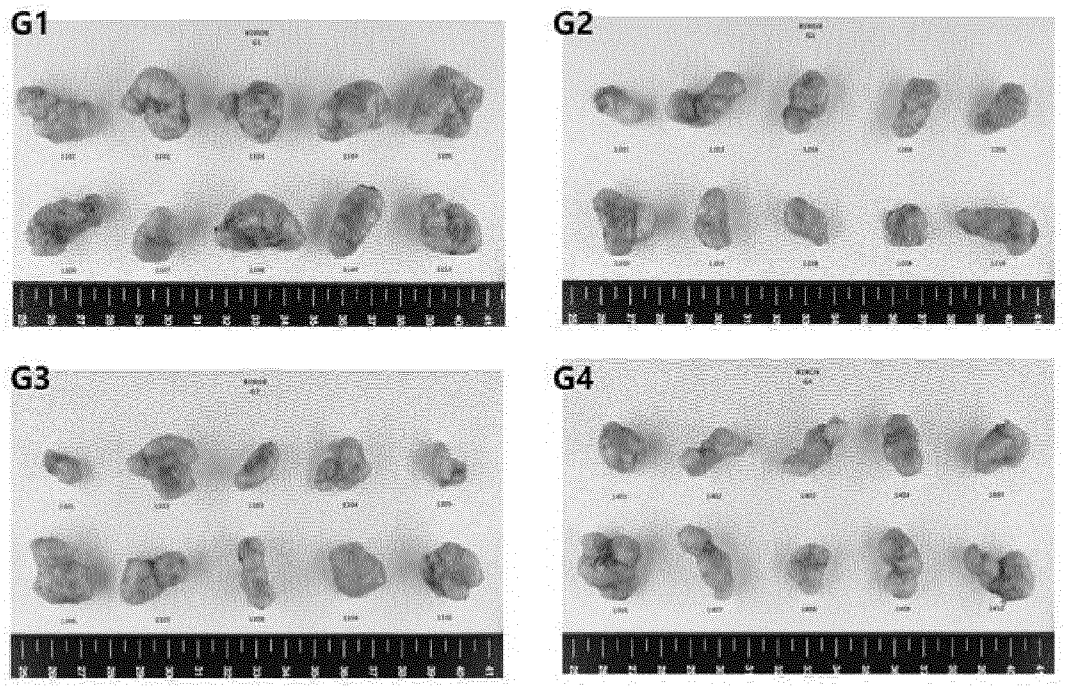

【FIG. 6e】

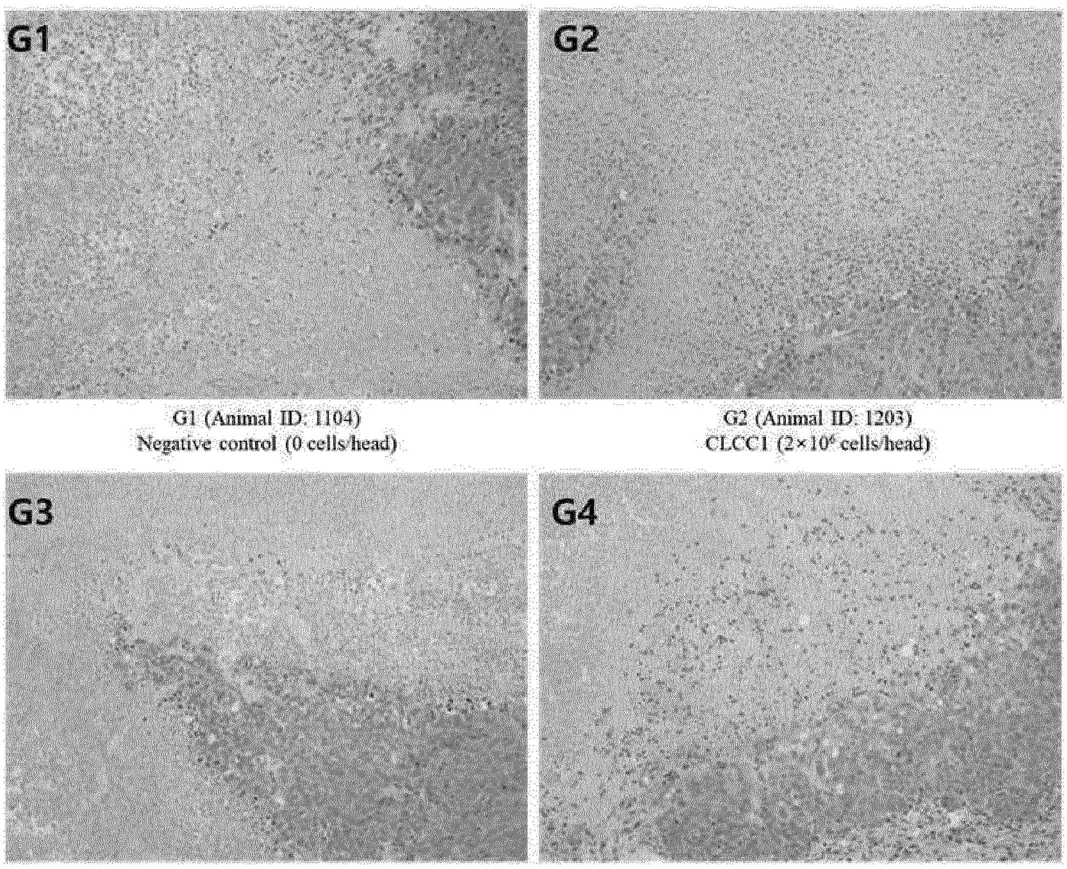


G1 (Animal ID: 1104)
Negative control (0 cells/head)

G2 (Animal ID: 1203)
CLCC1 ($2\times10^6$ cells/head)

G3 (Animal ID: 1308)
CLCC1 ($2\times10^7$ cells/head)

G4 (Animal ID: 1405)
CLCC1 ($2\times10^8$ cells/head)

【FIG. 6f】

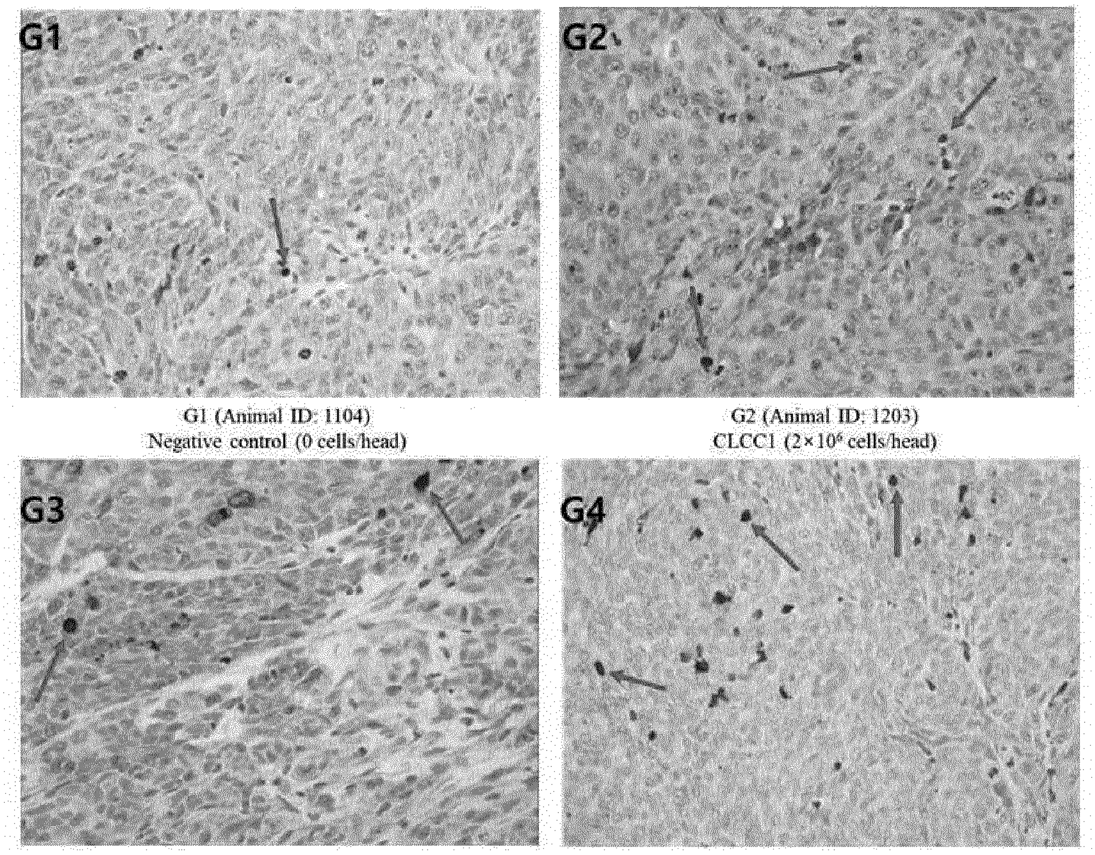


G1 (Animal ID: 1104)
Negative control (0 cells/head)

G2 (Animal ID: 1203)
CLCC1 ($2\times10^6$ cells/head)

G3 (Animal ID: 1308)
CLCC1 ($2\times10^7$ cells/head)

G4 (Animal ID: 1405)
CLCC1 ($2\times10^8$ cells/head)

【FIG. 7】

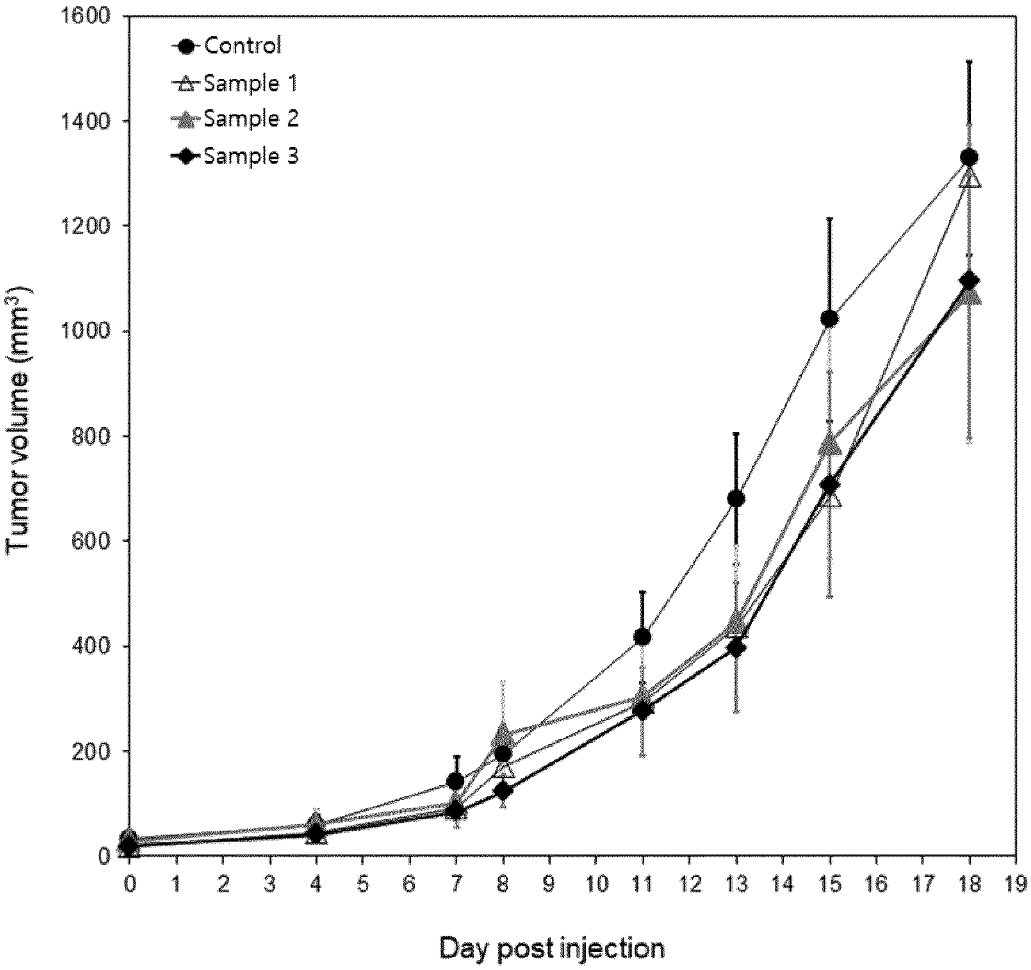
Control
Sample 1
Sample 2
Sample 3
Tumor volume (mm3)
Day post injection
0
200
400
600
800
1000
1200
1400
1600
0 1 2 3 4 5 6 7 8 9 10 11 12 13 14 15 16 17 18 19

【FIG. 8】

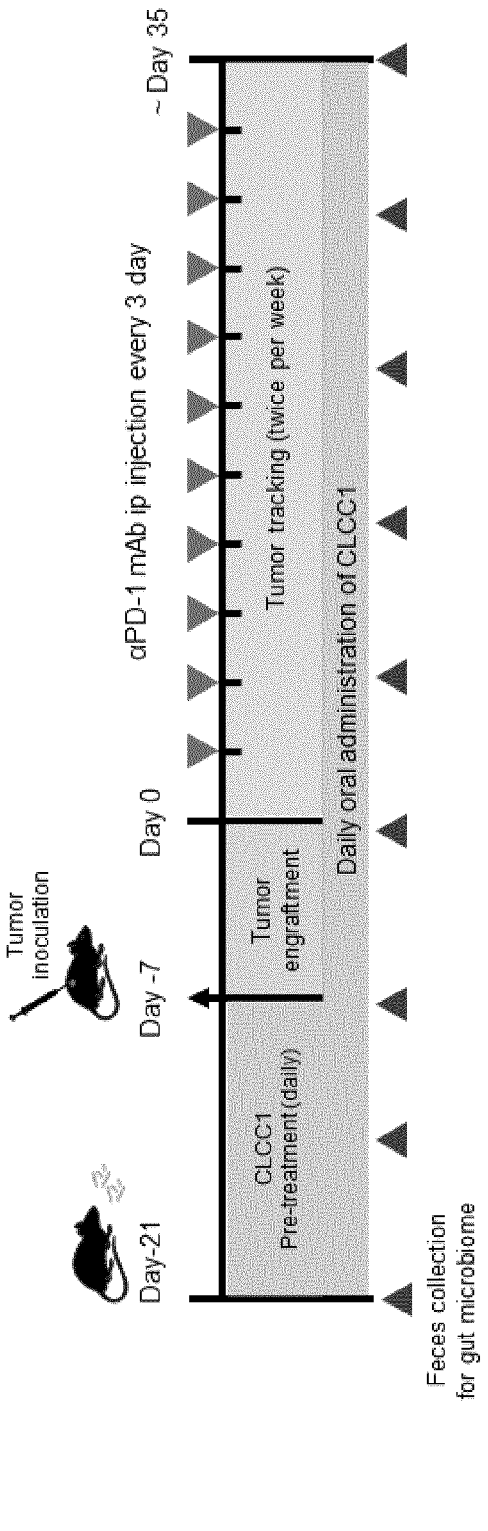
~ Day 35
αPD-1 mAb ip injection every 3 day
Tumor tracking (twice per week)
Daily oral administration of CLCC1
Day 0
Tumor engraftment
Tumor inoculation
Day -7
CLCC1 Pre-treatment (daily)
Day-21
Feces collection for gut microbiome

## INTERNATIONAL SEARCH REPORT

International application No.
PCT/KR2020/004363

A. CLASSIFICATION OF SUBJECT MATTER

*C12N 1/20(2006.01)i, A23L 33/105(2016.01)i, A61K 35/74(2006.01)i, A61P 35/00(2006.01)i, C12R 1/01(2006.01)n*

According to International Patent Classification (IPC) or to both national classification and IPC

B. FIELDS SEARCHED

Minimum documentation searched (classification system followed by classification symbols)
C12N 1/20; A61P 1/00; A23L 33/105; A61K 35/74; A61P 35/00; C12R 1/01

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: Faecalibacterium cancerinhibens, anti-cancer

C. DOCUMENTS CONSIDERED TO BE RELEVANT

| Category* | Citation of document, with indication, where appropriate, of the relevant passages | Relevant to claim No. |
|---|---|---|
| X | NCBI. GenBank accession no. MF185398.1 (08 June 2017) See DEFINITION; and the sequences. | 1-9 |
| Y | | 10-16 |
| Y | TIAN, Y. et al. Faecalibacterium prausnitzii prevents tumorigenesis in a model of colitis-associated colorectal cancer. AGA Abstracts. 01 April 2017, vol. 152, no. 5 (Suppl. 1), pages S354-S355, abstract Sa1810 See abstract Sa1810. | 10-16 |
| X | NCBI. GenBank accession no. MF185662.1 (08 June 2017) See DEFINITION; and the sequences. | 1-9 |
| Y | FERREIRA-HALDER, C. V. et al. Action and function of Faecalibacterium prausnitzii in health and disease. Best Practice & Research Clinical Gastroenterology. 2017, vol. 31, pages 643-648 See abstract. | 10-16 |
| A | WO 2018-027898 A1 (BGI SHENZHEN) 15 February 2018 See the entire document. | 1-16 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 JULY 2020 (16.07.2020) | **16 JULY 2020 (16.07.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2020/004363**

| Patent document cited in search report | Publication date | Patent family member | Publication date |
|---|---|---|---|
| WO 2018-027898 A1 | 15/02/2018 | CN 109874329 A | 11/06/2019 |
| | | EP 3498819 A1 | 19/06/2019 |
| | | EP 3498819 A4 | 08/04/2020 |
| | | US 2019-0240265 A1 | 08/08/2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MILLER, L. T.** *J. Clin. Microbiol.,* 1982, vol. 18, 861-867
- **MINIKIN, D.E et al.** *J Microbial Meth,* 1984, vol. 2, 233-241
- **TAKESHI FURUHASHI et al.** *Analytical Biochemistry,* 2018, vol. 543, 51-54

<110> ChunLab, Inc.

<120> Faecalibacterium sp. and anti-cancer composition comprising the same

<130> OPP20200807KR

<160> 1

<170> KoPatentIn 3.0

<210> 1
<211> 1499
<212> DNA
<213> Artificial Sequence

<220>
<223> Faecalibacterium cancerinhibens CLCC1 16S rRNA sequence

<400> 1
agagtttgat cctggctcag gacgaacgct ggcggcgcgc ctaacacatg caagtcgaac 60

gagcgagaga gagcttgctt tctcgagcga gtggcgaacg ggtgagtaac gcgtgaggaa 120

cctgcctcaa agagggggac aacagttgga aacgactgct aataccgcat aagcccacgg 180

ctcggcatcg agcagaggga aaaggagcaa tccgctttga gatggcctcg cgtccgatta 240

gctagttggt gaggtaatgg cccaccaagg cgacgatcgg tagccggact gagaggttga 300

acggccacat tgggactgag acacggccca gactcctacg ggaggcagca gtggggaata 360

ttgcacaatg gggaaaccc tgatgcagcg acgccgcgtg gaggaagaag gtcttcggat 420

tgtaaactcc tgttgttggg gaagataatg acggtaccca acaaggaagt gacggctaac 480

tacgtgccag cagccgcggt aaaacgtagg tcacaagcgt tgtccggaat tactgggtgt 540

aaagggagcg caggcgggaa gacaagttgg aagtgaaatc tatgggctca acccataaac 600

tgctttcaaa actgtttttc ttgagtagtg cagaggtagg cggaattccc ggtgtagcgg 660

tggaatgcgt agatatcggg aggaacacca gtggcgaagg cggcctactg ggcaccaact 720

gacgctgagg ctcgaaagtg tgggtagcaa acaggattag ataccctggt agtccacacc 780

gtaaacgatg attactaggt gttggaggat tgacccttc agtgccgcag ttaacacaat 840

aagtaatcca cctggggagt acgaccgcaa ggttgaaact caaaggaatt gacgggggcc 900

cgcacaagca gtggagtatg tggtttaatt cgacgcaacg cgaagaacct taccaagtct 960

tgacatccct tgacagacat agaaatatgt tttctcttcg gagcaaggag acaggtggtg 1020

catggttgtc gtcagctcgt gtcgtgagat gttgggttaa gtcccgcaac gagcgcaacc 1080

cttatggtca gttactacgc aagaggactc tggccagact gccgttgaca aaacggagga 1140

aggtggggat gacgtcaaat catcatgccc tttatgactt gggctacaca cgtactacaa 1200

tggcgttaaa caaagagaag caagaccgcg aggtggagca aaactcagaa acaacgtccc 1260

```
agttcggact gcaggctgca actcgcctgc acgaagtcgg aattgctagt aatcgtggat    1320
cagcatgcca cggtgaatac gttcccgggc cttgtacaca ccgcccgtca caccatgaga    1380
gccgggggga cccgaagtcg gtagtctaac cgcaaggagg acgccgccga aggtaaaact    1440
ggtgattggg gtgaagtcgt aacaaggtag ccgtaggaga acctgcggct ggatcacct     1499
```